(19) Europäisches Patentamt
European Patent Office
Office européen des brevets

(11) EP 3 282 218 A1

(12) EUROPEAN PATENT APPLICATION
published in accordance with Art. 153(4) EPC

(43) Date of publication:
14.02.2018 Bulletin 2018/07

(51) Int Cl.:
G01B 7/16 (2006.01)　A61B 5/11 (2006.01)

(21) Application number: 16776432.3

(22) Date of filing: 28.03.2016

(86) International application number:
PCT/JP2016/059954

(87) International publication number:
WO 2016/163264 (13.10.2016 Gazette 2016/41)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 06.04.2015 JP 2015077983

(71) Applicant: Bando Chemical Industries, Ltd.
Kobe-shi, Hyogo 650-0047 (JP)
(72) Inventors:
• OTAKA, Hideo
Kobe-shi
Hyogo 650-0047 (JP)
• OHTA, Masashi
Kobe-shi
Hyogo 650-0047 (JP)
• MATSUDA, Kazuaki
Kobe-shi
Hyogo 650-0047 (JP)

(74) Representative: Sajda, Wolf E.
Meissner Bolte Patentanwälte
Rechtsanwälte Partnerschaft mbB
Postfach 86 06 24
81633 München (DE)

(54) **CAPACITIVE SENSOR SHEET AND SENSOR DEVICE**

(57) The present invention relates to a capacitive sensor sheet for measuring the amount of deformation of a measuring object with high sensitivity and high accuracy. The capacitive sensor sheet of the present invention includes a sensor body; and a stretchable cloth integrally formed with the sensor body and having anisotropy in terms of stretchability, the sensor body including: a sheet-like dielectric layer including an elastomer composition; and a first electrode layer and a second electrode layer each including an electroconductive composition containing an electroconductive material, wherein the first and the second electrode layers are formed on a top surface and a bottom surface of the dielectric layer respectively, and are at least partially opposed to each other across the dielectric layer, the at least partially opposed portions of the first and the second electrode layers constitute a detection portion, and the sensor body is reversibly deformable to change areas of the top and bottom surfaces of the dielectric layer.

FIG. 3A

FIG. 3B

**Description**

TECHNICAL FIELD

The present invention relates to a capacitive sensor sheet and a sensor device.

BACKGROUND ART

In the field of rehabilitation, measurements have been conducted on a daily basis about, for example, the momentum of a patient in the state of a motor paralysis, such as partial paralysis or hemiplegia, or the movable quantity or movable range of, for example, his/her joint, or about the heart rate or respiratory rate of a patient when the patient is in training or is staying in bed.

In the medical field also, the heart rate or respiratory rate of a patient or an elderly person who requires nursing has been monitored on a daily basis.

As a method for measuring the momentum of a patient or the movable quantity or movable range of, for example, his/her joint, the following methods have been conventionally adopted: a method using a ruler, a method using a goniometer, and a method using a myoelectric sensor.

However, although these methods make it possible to measure the degree of a bending of his/her elbow joint and knee joint, there are a large number of his/her regions which are not easily measured by the methods, such as his/her shoulder bone motion, his/her buttock motion, and his/her expression motion.

Furthermore, as a method for measuring a larger motion of a body, suggested is also a method using a motion capture (see, for example, Patent Literature 1). However, the motion-capture-using method requires a large system as a whole of a measuring system; thus, it is difficult to carry the measuring system portably. Additionally, it is complicated to make a preparation before the measurement, and further this method has a problem of making it impossible to measure the motion of a region behind a photographing device (camera).

Furthermore, as a method for monitoring the heart rate or respiratory rate of, for example, a patient with the passage of time, for example, Patent Literature 2 suggests a method using a capacitive pressure sensor formed by locating a pair of stretchable electroconductive clothes, respectively, on both surfaces of a sheet-like dielectric elastically deformable in all directions.

However, according to the method described in Patent Literature 2, a person is not easily measured in the state of moving his/her body, for example, at the time of being in rehabilitation training since the capacitive pressure sensor is laid below a human body lying down and then the heart rate or respiratory rate of the person is monitored.

Moreover, the capacitive pressure sensor is a sensor for measuring a change in capacitance of a portion of the sensor by a deformation of its dielectric layer in the thickness direction thereof. Accordingly, a deformation of the dielectric layer in a surface direction is not originally measured with ease to make it impossible to measure the motion state of the body.

LIST OF CITATIONS

PATENT LITERATURE

Patent Literature 1: WO 2005/096939 A

Patent Literature 2: JP 2005-315831 A

SUMMARY OF INVENTION

TECHNICAL PROBLEM

Under such situations, the inventors of the present invention have suggested a capacitive sensor device based on the technical idea different from conventional capacitive sensor devices, as a capacitive sensor device capable of tracing a deformation of a surface of a living body.

That is, the inventors of the present invention have suggested a sensor device including:

a sensor element; and
a measuring instrument measuring a change in capacitance of the detection portion, the sensor element including

a sheet-like first dielectric layer including an elastomer composition; and

a first electrode layer and a second electrode layer each including an electroconductive composition containing carbon nanotubes,

wherein the first and second electrode layers are formed on a top surface and a bottom surface of the first dielectric layer respectively.

In the sensor device, the first and second electrode layers are at least partially opposed to each other across the dielectric layer, and the at least partially opposed portions of the first and the second electrode layers constitute a detection portion, and the sensor body is reversibly deformable to change areas of the top and bottom surfaces of the dielectric layer.

The sensor element in such a sensor device includes a dielectric layer including an elastomer composition and an electroconductive layer including an electroconductive composition containing carbon nanotubes and reversibly deforms to change areas of the top and bottom surfaces of the dielectric layer.

Therefore, for example, in a case where the sensor element is attached to a living body to follow a deformation of the living body surface, even when the living body surface is largely deformed, the amount of deformation can be measured as the amount of change of capacitance. Accordingly, in such a sensor device, the motion state of the body can be measured and measurement can be carried out even in the state of moving his/her body, for example, at the time of rehabilitation training.

However, there is a demand for improvement in sensitivity and accuracy of such a sensor element, as described below.

Generally, capacitance in a capacitive sensor is represented by the following Formula (1):

$$C = \varepsilon_0 \varepsilon_r S/d \qquad \ldots(1).$$

Here, C represents capacitance; $\varepsilon_0$ represents the permittivity of free space; $\varepsilon_r$ represents the relative permittivity of a dielectric layer; S represents the area of an electrode layer; and d represents the distance between electrodes.

In a case where the sensor element deforms in the surface direction (deforms to change the areas of the top and bottom surfaces of the dielectric layers), the electroconductive layer including an electroconductive composition containing carbon nanotubes is deformed to follow the deformation of the dielectric layer (that is, the value of S in the above Formula (1) changes), and as a result, the value of the capacitance C changes. Therefore, the sensor element can detect the amount of deformation of the sensor element on the basis of the amount of change $\Delta C$ of the capacitance C.

On the other hand, in a case where the dielectric layer is elongated in a uniaxial direction, the electroconductive layer is also elongated in the same direction according to the elongation of the dielectric layer and the area thereof is increased. At this time, the dielectric layer may be shrunk also in a direction (width direction) perpendicular to an elongation direction according to the elongation of the dielectric layer. In this case, the electroconductive layer may also be shrunk in the direction (width direction) perpendicular to the elongation direction so as to follow shrinkage of the dielectric layer in the width direction.

When such shrinkage of the electroconductive layer in the width direction occurs, correlativity between the elongation amount of the dielectric layer (electroconductive layer) and the increase amount of the area is decreased. As a result, measurement sensitivity and measurement accuracy of the sensor element are decreased.

For these reasons, as described above, there is a demand for improvement in sensitivity and accuracy.

SOLUTION TO PROBLEM

The inventors of the present invention have conducted intensive studies to respond to such a demand, and thus have completed the present invention.

A capacitive sensor sheet of the present invention includes:

a sensor body; and

a stretchable cloth integrally formed with the sensor body and having anisotropy in terms of stretchability, the sensor body including:

a sheet-like dielectric layer including an elastomer composition; and

a first electrode layer and a second electrode layer each including an electroconductive composition containing an electroconductive material,

wherein the first and the second electrode layers are formed on a top surface and a bottom surface of the dielectric layer respectively, and are at least partially opposed to each other across the dielectric layer, the at least partially opposed portions of the first and the second electrode layers constitute a detection portion, and the sensor body is reversibly deformable to change areas of the top and bottom surfaces of the dielectric layer.

The capacitive sensor sheet of the present invention has a cloth integrally formed with a sensor body, and the cloth has anisotropy in terms of stretchability. For this reason, in a case where the sensor body is elongated in an easily-stretchable direction of the cloth, the electrode layer is elongated in the elongation direction but is hardly elongated in a direction (width direction) perpendicular to the elongation direction.

Accordingly, when the sensor body is elongated, the area of the electrode layer is increased in proportion to the elongation amount of the sensor body. As a result, in the sensor sheet of the present invention, regardless of the elongation rate of the sensor body, the proportional relationship between the elongation amount of the sensor body and the capacitance of the detection portion is maintained.

In the capacitive sensor sheet of the present invention, it is preferable that the cloth has a ratio of modulus at 5 % in a poorly-stretchable direction to modulus at 5 % in an easily-stretchable direction ([M5 in the poorly-stretchable direction] / [M5 in the easily-stretchable direction]) of 10 or more.

In the capacitive sensor sheet of the present invention, it is preferable that the electroconductive material is at least carbon nanotubes.

Furthermore, in the capacitive sensor sheet of the present invention, it is preferable that the elastomer composition contains urethane elastomer.

A sensor device of the present invention includes the capacitive sensor sheet of the present invention and a measuring instrument measuring a change in capacitance of the detection portion.

Since the sensor device of the present invention includes the capacitive sensor sheet of the present invention, the amount of deformation of a measuring object can be accurately measured on the basis of the amount of change of capacitance of the sensor sheet.

## ADVANTAGEOUS EFFECTS OF INVENTION

According to the present invention, it is possible to provide a capacitive sensor sheet and a sensor device for measuring the amount of deformation of a measuring object with high sensitivity and high accuracy.

## BRIEF DESCRIPTION OF DRAWINGS

| | |
|---|---|
| FIG. 1 | is a schematic diagram illustrating an example of a sensor device of the present invention. |
| FIG. 2A | is a perspective view schematically illustrating an example of a sensor body that constitutes a capacitive sensor sheet of the present invention, and |
| FIG. 2B | is a cross-sectional view obtainable by cutting the sensor body along the line A-A shown in FIG. 2A. |
| FIG. 3A | is a perspective view schematically illustrating an example of the capacitive sensor sheet of the present invention, and FIG. 3B is a cross-sectional view obtainable by cutting the capacitive sensor sheet along the line B-B shown in FIG. 3A. |
| FIG. 4A | is a perspective view schematically illustrating another example of the sensor body that constitutes the capacitive sensor sheet of the present invention, and |
| FIG. 4B | is a cross-sectional view obtainable by cutting the sensor body along the line C-C shown in FIG. 4A. |
| FIG. 5 | is a schematic diagram for explaining an example of a forming apparatus used for the production of a dielectric layer that is included in the capacitive sensor sheet of the present invention. |
| FIGS. 6A to 6C | are perspective views for explaining production processes for a sensor body in Examples. |
| FIGS. 7A to 7D | are perspective views for explaining production processes for a capacitive sensor sheet in Examples. |
| FIG. 8 | is a graph showing measurement results of capacitance when a capacitive sensor sheet of Example 1 is subjected to a reciprocatory elongation operation. |
| FIG. 9 | is a graph showing measurement results of capacitance when a capacitive sensor sheet of Example 2 is subjected to a reciprocatory elongation operation. |
| FIG. 10 | is a graph showing measurement results of capacitance when a capacitive sensor sheet of Comparative Example 1 is subjected to a reciprocatory elongation operation. |
| FIG. 11 | is a graph showing measurement results of capacitance when a capacitive sensor sheet of Comparative Example 2 is subjected to a reciprocatory elongation operation. |
| FIG. 12 | is a graph showing measurement results of capacitance when a capacitive sensor sheet of Comparative Example 3 is subjected to a reciprocatory elongation operation. |
| FIG. 13 | is a graph showing measurement results of capacitance when a capacitive sensor sheet of Compar- |

ative Example 4 is subjected to a reciprocatory elongation operation.

DESCRIPTION OF EMBODIMENTS

Hereinafter, embodiments of the present invention will be described with reference to the drawings.

A capacitive sensor sheet of the present invention (hereinafter, also simply referred to as the sensor sheet) includes: a sensor body; and a stretchable cloth integrally formed with the sensor body and having anisotropy in terms of stretchability, the sensor body including: a sheet-like dielectric layer including an elastomer composition; and a first electrode layer and a second electrode layer each including an electroconductive composition containing an electroconductive material, wherein the first and the second electrode layers are formed on a top surface and a bottom surface of the dielectric layer respectively, and are at least partially opposed to each other across the dielectric layer, the at least partially opposed portions of the first and the second electrode layers constitute a detection portion, and the sensor body is reversibly deformable to change areas of the top and bottom surfaces of the dielectric layer.

A sensor device of the present invention includes the capacitive sensor sheet of the present invention and a measuring instrument measuring a change in capacitance of the detection portion.

FIG. 1 is a schematic diagram illustrating an example of a sensor device of the present invention, and the sensor device includes the capacitive sensor sheet of the present invention.

FIG. 2A is a perspective view schematically illustrating an example of a sensor body that constitutes a capacitive sensor sheet of the present invention, and FIG. 2B is a cross-sectional view obtainable by cutting the sensor body along the line A-A shown in FIG. 2A.

FIG. 3A is a perspective view schematically illustrating an example of the capacitive sensor sheet including the sensor body illustrated in FIGS. 2A and 2B, and FIG. 3B is a cross-sectional view obtainable by cutting the capacitive sensor sheet along the line B-B shown in FIG. 3A.

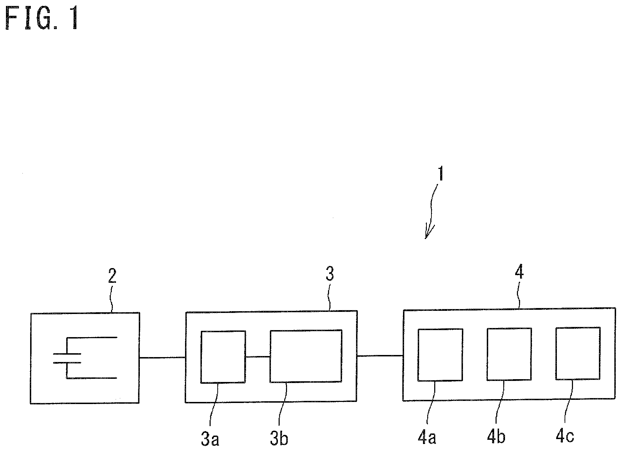

As illustrated in FIG. 1, a sensor device 1 according to the present invention includes a sensor sheet 2 according to the present invention, a measuring instrument 3 that is electrically connected to the sensor sheet 2, and a display device 4 for displaying the measurement results obtained in the measuring instrument 3.

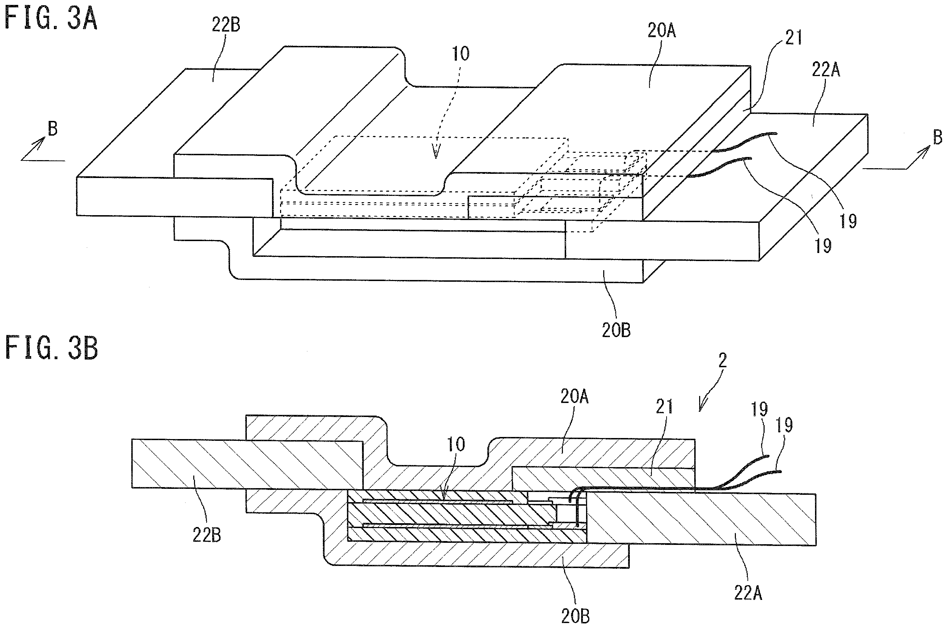

As illustrated in FIGS. 3A and 3B, the sensor sheet 2 includes a sensor body 10 and clothes 20A and 20B that are laminated on both surfaces (the top surface and the bottom surface) of the sensor body 10 and have anisotropy in terms of stretchability.

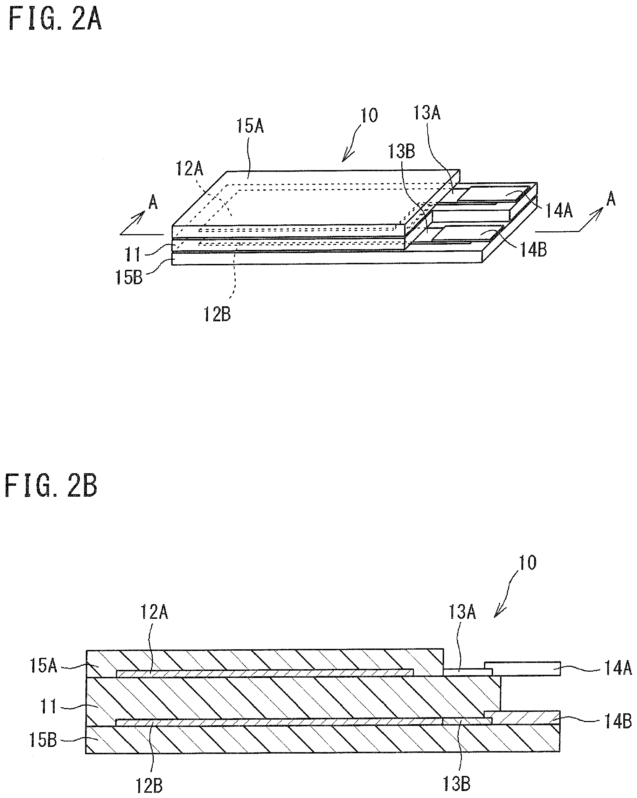

As illustrated in FIGS. 2A and 2B, the sensor body 10 includes a sheet-like dielectric layer 11 composed of an elastomer composition; a top electrode layer 12A formed on the top surface of the dielectric layer 11; a bottom electrode layer 12B formed on the bottom surface of the dielectric layer 11; a top conducting wire 13A connected to the top electrode layer 12A; a bottom conducting wire 13B connected to the bottom electrode layer 12B; a top connecting portion 14A mounted at the end of the top conducting wire 13A that is on the opposite side of the top electrode layer 12A; a bottom connecting portion 14B mounted at the end of the bottom conducting wire 13B that is on the opposite side of the bottom electrode layer 12B; a top protective layer 15A laminated on the top side of the dielectric layer 11; and a bottom protective layer 15B laminated on the bottom side of the dielectric layer 11.

Herein, the top electrode layer 12A and the bottom electrode layer 12B have the same planar view shape and are entirely opposed to each other across the dielectric layer 11. In the sensor body 10, a portion where the top electrode layer 12A and the bottom electrode layer 12B are opposed to each other constitutes a detection portion.

In the present invention, it is not necessarily required that the top electrode layer and the bottom electrode layer in the sensor body are entirely opposed to each other across the dielectric layer, and these electrode layers are sufficient to be at least partially opposed to each other.

In the sensor body 10, the top electrode layer 12A corresponds to the first electrode layer and the bottom electrode layer 12B corresponds to the second electrode layer.

In the sensor body 10, since the dielectric layer 11 is composed of the elastomer composition, the dielectric layer 11 can be deformed (stretched) in a surface direction. When the dielectric layer 11 is deformed in the surface direction, the top electrode layer 12A, the bottom electrode layer 12B, and the top protective layer 15A and the bottom protective layer 15B (hereinafter, the two may also be together simply referred to as protective layers) are deformed in conformity with the deformation of the dielectric layer 11.

Along with the deformation of the sensor body 10, the capacitance of the detection portion changes with a correlation between the capacitance and the amount of deformation of the dielectric layer 11 (change in area of the electrode layer). Accordingly, by detecting the change in capacitance of the detection portion, the amount of deformation of the sensor sheet 2 can be detected.

Further, since the sensor sheet 2 includes a cloth to be described below, the sensor device 1 can substantially detect the amount of deformation of the sensor body 10 in the uniaxial direction by detecting the change in capacitance of the detection portion.

The clothes 20A and 20B are laminated on the top side and the bottom side of the sensor body 10 respectively through an adhesive layer (not shown in the diagram).

Both the clothes 20A and 20B are a cloth that has anisotropy in terms of stretchability. The clothes 20A and 20B are laminated so as to cover the entirety of the detection portion when the sensor sheet 2 is viewed in a planar view. Further, the clothes 20A and 20B are laminated such that a longitudinal direction (the right-left direction in the drawing) of the sensor body 10 becomes the easily-stretchable direction and a direction perpendicular to the longitudinal direction becomes the poorly-stretchable direction.

The sensor sheet 2 includes a non-stretchable member 21 and handles 22A and 22B.

These are arbitrary members, and it is not necessarily required that the capacitive sensor sheet of the present invention includes these members.

The non-stretchable member 21 is laminated on the upper surface of the sensor body 10. The non-stretchable member 21 is laminated at a position that does not overlap with the detection portion when the sensor sheet 2 is viewed in a planar view and covers at least the top conducting wire 13A, the bottom conducting wire 13B, the top connecting portion 14A, and the bottom connecting portion 14B.

In the sensor sheet 2 including the non-stretchable member 21, when the sensor sheet 2 is stretched and/or shrunk, the detection portion of the sensor body 10 is stretched and/or shrunk and the portion covered with the non-stretchable member 21 of the sensor body 10 is difficult to stretch or shrink. For this reason, stretch/shrinkage of the detection portion is more accurately reflected by the deformation of the measuring object. Therefore, the sensor device 1 has more excellent measurement sensitivity owing to the non-stretchable member 21 of the sensor sheet 2.

The handles 22A and 22B are disposed to be positioned at both ends in the easily-stretchable direction of the sensor body 10. The handles 22A and 22B are interposed with two clothes 20A and 20B.

The handles 22A and 22B have roles of holding the sensor sheet 2 by a user when the sensor sheet 2 is stretched and/or shrunk and fixing the sensor sheet to a predetermined position.

In the sensor sheet 2, the clothes 20A and 20B have anisotropy in terms of stretchability. For this reason, when the sensor body 10 is elongated in the longitudinal direction, it is possible to suppress the shrinkage of the sensor body 10 in the direction (width direction) perpendicular to the longitudinal direction as the elongation amount increases. As a result, the capacitance of the detection portion of the sensor sheet 2 is increased according to the elongation amount of the sensor body 10, and the correlation (proportional relationship) between the elongation amount and the capacitance is also maintained in a case where the elongation amount of the sensor body 10 is increased or in a case where the sensor body 10 is repeatedly stretched and/or shrunk. Therefore, the sensor device 1 can measure the elongation amount of the sensor body 10 with high sensitivity and high accuracy.

The measuring instrument 3 includes a Schmitt trigger oscillator circuit 3a for converting capacitance C to a frequency signal F; a F/V conversion circuit 3b that converts the frequency signal F to a voltage signal V; and a power supply circuit (not shown in the drawing). The measuring instrument 3 converts capacitance C detected in the detection portion of the sensor sheet 2 to a frequency signal F, subsequently further converts the frequency signal F to a voltage signal V, and transmits the voltage signal V to the display device 4. As will be described below, the configuration of the measuring instrument 3 is not intended to be limited to such a configuration.

The display device 4 includes a monitor 4a; an arithmetic circuit 4b; and a memory unit 4c. The display device 4 displays the change in capacitance C of the detection portion measured at the measuring instrument 3, on the monitor 4a, and also stores the change in capacitance C as recorded data.

Further, the display device 4 may calculate the amount of deformation of a measuring object on the basis of the voltage signal V received from the measuring instrument 3 with the arithmetic circuit 4b and display the amount of deformation of the measuring object on the monitor 4a.

As the display device 4, a computer including a memory unit such as a CPU, a RAM, a ROM, or a HDD, a monitor, various input/output interfaces, and the like can be used.

In such a sensor device 1, the sensor sheet 2 can be used in a state of being attached to a measuring object.

For this reason, an adhesive layer for attaching the sensor sheet 2 to a measuring object may be formed on the outermost layer of the sensor sheet 2.

The sensor device 1 can follow a deformation of the living body surface, for example, by using the sensor sheet 2 in a state of being attached to the living body surface. At this time, the sensor sheet 2 may be attached directly to the living body surface, or may be attached indirectly to the living body surface to interpose, therebetween, a covering member, which covers the living body surface, such as clothing, a supporter, or a bandage.

In a case where the sensor sheet 2 is used in a state of being attached to the living body surface such as skin, the sensor sheet follows a deformation (such as elongation/withering, or swelling/contraction) of the living body surface to be stretched and/or shrunk. Thus, the capacitance of the detection portion is changed in accordance with the amount of deformation of the living body surface.

For this reason, by measuring the capacitance of the detection portion, the sensor device 1 can measure the amount of deformation of the living body surface. Further, by measuring the amount of deformation of the living body surface, information pieces can be achieved on biological activities of the living body that are correlative with the deformation of the living body surface, as well as on motions of the living body.

The sensor device of the present invention makes it possible to measure, as the biological activity information pieces, for example, the pulse rate (heart rate), the respiratory rate, and the level of respirations of any living body.

The motion information pieces of the living body are not particularly limited. As long as the motion of the living body is such a motion that the living body surface is stretched and/or shrunk by the stretch and/or shrinkage of its muscle when the living body moves, the sensor device can measure the state of the motion.

Specifically, examples of the motion information pieces of the living body which can be measured include the bending quantity (bending angle) of a joint when the joint is bent, a cheek motion when he/she pronounces or speaks, a motion of muscle of facial expression, a shoulder bone motion, a gluteal muscle motion, a back motion, the bending degree of waist, the swelling of breast, the degree of the contraction of thigh or calf by the contraction of its muscle, a throat motion when swallowing, a leg motion, a hand motion, a finger motion, a foot sole motion, a wink motion, and the stretchability (softness) of skin.

When the sensor device is used to measure a pulse rate (heart rate) of a living body, the heart rate can be achieved by attaching the sensor sheet to a surface region of the living body where pulses are felt (the region is, for example, a radial artery or a carotid artery), and then continuing to measure the capacitance over a predetermined period. This is because the skin stretches and shrinks in accordance with the pulses, and the number of the stretches/shrinkages is consistent with the number of the pulses.

When the sensor device is used to measure a respiratory rate of a living body, the respiratory rate can be achieved, by attaching the sensor sheet to a breast region or some other region of a surface of the living body, and then continuing to measure the capacitance over a predetermined period. This is because the skin of the breast region stretches and shrinks in accordance with the respiration, and the number of the stretches/shrinkages is consistent with the number of the respirations.

When the sensor device is used to measure the bending quantity of a joint, the bending quantity of the region to be measured can be achieved by attaching the sensor sheet to the region to be measured, and then measuring the capacitance while the region to be measured is moved. This is because the skin of the region is stretched and/or shrunk in accordance with the movement of the region to be measured so that the bending quantity of the region to be measured can be calculated in accordance with the quantity of the stretch/shrinkage.

Further, the sensor sheet is attached to a region around a mouth (for example, his/her cheek) and the capacitance is measured while in this state, he/she speaks (or he/she tries to speak even when he/she cannot actually speak). At this time, the skin around his/her mouth is deformed in accordance with the kind of the spoken sound. Thus, in accordance with the deformation of the skin, the capacitance comes to be changed. It is therefore possible to achieve correlation information between the motion of the skin around his/her mouth when he/she speaks and the value of the capacitance or the changing manner of the value.

In this way, for example, the following training can be attained.

For his/her muscle training of facial expression, sensor sheets are bisymmetrically attached to him/her, whereby the motion of his/her skin can be quantitatively measured or can be made visible in real time. As a result, while viewing signal waveforms from the right and left sides, he/she can be in training to superpose these signals consciously onto each other or he/she can be in rehabilitation training for recovering his/her function to show a bisymmetrical and natural facial expression.

The sensor sheet is attached to an ankle or instep, and the capacitance is measured while in this state, he/she is taking an exercise, such as "stamping of his/her feet", "jumping", "standing-up on tiptoes", or "standing-still". At this time, in accordance with the motion of the leg or foot, skin is deformed. In accordance with the deformation of the skin, the capacitance comes to be changed. Thus, on the basis of the value of the capacitance or the changing manner of the value, the motion of the leg or foot can be specified.

The sensor sheet is attached to the palm or the back of a hand, and the capacitance is measured while in this state, he/she is taking an exercise, such as "opening of his/her hand", "closing of his/her hand", "a raise of any finger" or "playing of rock-paper-scissors". At this time, in accordance with the motion of the hand, skin is deformed. In accordance with the motion of the skin, the capacitance comes to be changed. Thus, on the basis of the value of the capacitance, or the changing manner of the value, the motion of the hand can be specified.

According to the use of the sensor sheet in the state of being attached to the living body surface such as skin, the sensor device of the present invention can measure various biological activity information pieces and living body motion information pieces.

When the sensor device is used to measure any information piece on a biological activity or a motion of any one out of plural living bodies, it is preferred to achieve, as a proofreading information piece beforehand, a relationship between the kind of the motion and the value of the capacitance or the changing manner of the value for each of the living bodies to be measured. This is because a more precise measurement can be made even when the living bodies have a difference between any two thereof.

In the case of using the sensor sheet 2 in the state of being attached to a living body to interpose a covering member such as clothing or a supporter therebetween, the deformation information of the covering member can be measured.

For example, when a person attaches the sensor sheet 2 to his/her underwear for training, and in this state, he/she takes an exercise, the cloth texture of the underwear for training follows the motion of his/her body to be stretched or recovered into an original state thereof. Thus, the cloth texture is deformed. Accordingly, in accordance with the deformation (stretch and shrinkage) of the cloth texture, the capacitance comes to be changed. Thus, on the basis of the value of the capacitance, or the changing manner of the value, the sensor device can measure the deformation of the underwear for training (covering member).

The sensor device of the present invention may include a plurality of sensor sheets. In this case, the sensor device may achieve information pieces of the same kind simultaneously at different regions of a living body, or may achieve information pieces of different kinds simultaneously.

Further, in a case where the sensor device includes two or more sensor sheets, for example, the sensor sheets are bisymmetrically attached to a body (for example, the instep of his/her right foot and that of his/her left foot) and in this state, he/she stamps his/her feet. In this way, the balance between the respective motions of his/her right and left legs can be measured.

Further, for example, when a person attaches the sensor sheets, respectively, to his/her right and left ankles and knee joints, and hip joint and then walks in this state, measurement can be carried out about the balance between the respective motions of his/her right and left legs, the bending quantity of each of his/her mobile regions, and the motion rhythm of each of his/her mobile regions. Furthermore, when he/she uses the sensor device together with an existing walking measurement instrument such as a pressure distribution sensor product for a shoe shape or a mat shape, he/she can achieve higher-level information pieces on his/her walking motion.

These information pieces are useful for information pieces for deciding a menu of sports training or rehabilitation training.

The method of using the sensor device of the present invention is not limited to the method of attaching the sensor sheet to the living body surface. The sensor device can also measure the deformation of a measuring object by using a stretch/shrinkage object such as an expander, a rehabilitation tube, a rubber ball, a rubber balloon, or an air bag, a flexible object such as a cushion or a sole inner, or the like as a measuring object and attaching the sensor sheet to the measuring object.

In the sensor device of the present invention, the sensor sheet is usable as an alternate product for an interface of a myoelectric sensor for an electrically-powered artificial hand or leg.

Further, in the sensor device of the present invention, the sensor sheet is usable also as an inputting terminal of an inputting interface for a serious psychosomatic handicapped person.

Further, in the sensor device of the present invention, by attaching the sensor sheet to a finger portion of a glove, this glove is usable also as a glove type interface of virtual equipment, or the like.

In the capacitive sensor sheet of the present invention, the sensor body may include a second dielectric layer and a third electrode layer in addition to the dielectric layer (first dielectric layer) and the first and second electrode layers formed on both surfaces of the dielectric layer.

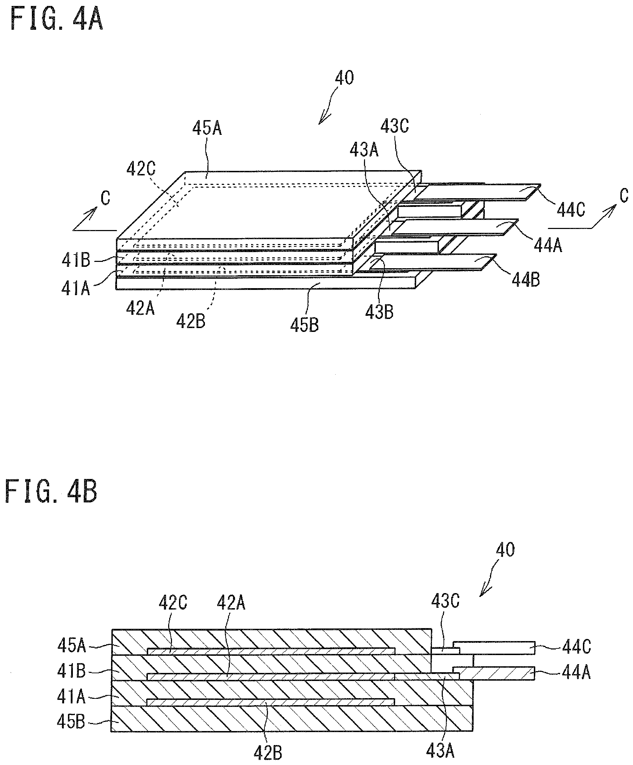

FIG. 4A is a perspective view schematically illustrating another example of the sensor body that constitutes the capacitive sensor sheet of the present invention, and FIG. 4B is a cross-sectional view obtainable by cutting the sensor body along the line C-C shown in FIG. 4A.

A sensor body 40 illustrated in FIGS. 4A and 4B includes a sheet-like first dielectric layer 41A composed of an elastomer composition; a first electrode layer 42A formed on the top surface of the first dielectric layer 41A; a second electrode layer 42B formed on the bottom surface of the first dielectric layer 41; a second dielectric layer 41B laminated on the top side of the first dielectric layer 41A so as to cover the first electrode layer 42A; and a third electrode layer 42C formed on the top surface of the second dielectric layer 41B.

Further, the sensor body 40 includes a first conducting wire 43A connected to the first electrode layer 42A; a second conducting wire 43B connected to the second electrode layer 42B; a third conducting wire 43C connected to the third electrode layer 42C; a first connecting portion 44A mounted at the end of the first conducting wire 43A that is on the opposite side of the first electrode layer 42A; a second connecting portion 44B mounted at the end of the second conducting wire 43B that is on the opposite side of the second electrode layer 42B; and a third connecting portion 44C mounted at the end of the third conducting wire 43C that is on the opposite side of the third electrode layer 42C. Furthermore, the sensor body 40 includes a bottom protective layer 45B on the bottom side of the first dielectric layer 41A and a top protective layer 45A on the top side of the second dielectric layer 41B.

The first electrode layer 42A to the third electrode layer 42C have the same planar view shape. The first electrode layer 42A and the second electrode layer 42B are entirely opposed to each other across the first dielectric layer 41A, and the first electrode layer 42A and the third electrode layer 42C are entirely opposed to each other across the second dielectric layer 41B.

In the sensor body 40, a detection portion is not only a portion where the first electrode layer 42A and the second electrode layer 42B are opposed to each other, but also a portion where the first electrode layer 42A and the third electrode layer 42C are opposed to each other. The capacitance of the detection portion is the sum of the capacitance of the portion where the first electrode layer 42A and the second electrode layer 42B are opposed to each other, and the capacitance of the portion where the first electrode layer 42A and the third electrode layer 42C are opposed to each other.

A sensor sheet having such a sensor body 40 excludes measurement accidental errors based on noises and thus is suitable for more precisely measuring a change in the capacitance.

About this matter, description will be made in some more detail. In a case where the deformation of the living body surface is followed by using the sensor device of the present invention, when measurement is carried out using a sensor body having one dielectric layer and electrode layers formed on both surfaces thereof or a sensor body further having a protective layer like the sensor body 10, the contact or approach of the living body surface to the electrode layer causes noises since the living body surface is an electroconductor.

Against this problem, in the sensor device including the sensor sheet having the sensor body 40, measurement accidental errors based on noises can be more effectively excluded. Further, the sensor sheet having the configuration illustrated in FIGS. 4A and 4B can be used without any discrimination between the top surface and the bottom surface.

Hereinafter, various members that are included in the capacitive sensor sheet of the present invention will be described in detail.

Sensor sheet

Dielectric layer

The dielectric layer is a sheet-like product including an elastomer composition and can reversibly deform to change areas of the top and bottom surfaces thereof. In the present invention, the top and bottom surfaces of the sheet-like dielectric layer mean the top surface and the bottom surface of the dielectric layer.

The elastomer composition may be a composition including an elastomer and other optional components as necessary.

Examples of the elastomer include natural rubber, isoprene rubber, nitrile rubber (NBR), ethylene-propylene rubber (EPDM), styrene-butadiene rubber (SBR), butadiene rubber (BR), chloroprene rubber (CR), silicone rubber, fluorine rubber, acrylic rubber, hydrogenated nitrile rubber, and urethane elastomer. These may be used singly, or two or more kinds thereof may be used in combination.

Among these, urethane elastomer and silicone rubber are preferable. It is because these rubbers have small permanent strain (or permanent elongation). Furthermore, urethane elastomer is particularly preferred because urethane elastomer has excellent adhesion to carbon nanotube compared to silicone rubber.

The urethane elastomer is a product obtainable as a result of a reaction between at least a polyol component and an isocyanate component. Specific examples of the urethane elastomer include, for example, an olefin-based urethane elastomer containing an olefin-based polyol as a polyol component; an ester-based urethane elastomer containing an ester-based polyol as a polyol component; an ether-based urethane elastomer containing an ether-based polyol as a polyol component; a carbonate-based urethane elastomer containing a carbonate-based polyol as a polyol component; and a castor oil-based urethane elastomer containing a castor oil-based polyol as a polyol component.

These may be used singly, or two or more kinds thereof may be used in combination. The urethane elastomer may be a product obtained by using two or more kinds of the above-mentioned polyol components in combination.

Examples of the olefin-based polyol include EPOL (manufactured by Idemitsu Kosan Co., Ltd.).

Examples of the ester-based polyol include POLYLITE 8651 (manufactured by DIC Corp.).

Examples of the ether-based polyol include polyoxytetramethylene glycol, PTG-2000SN (manufactured by Hodogaya Chemical Co., Ltd.), polypropylene glycol, PREMINOL S3003 (manufactured by Asahi Glass Co., Ltd.), and PANDEX GCB-41 (manufactured by DIC Corp.).

The isocyanate component is not particularly limited, and any conventionally known isocyanate component can be used.

When the urethane elastomer is synthesized, a chain extending agent, a crosslinking agent, a catalyst, a vulcanization accelerator, and the like may be added to the reaction system, as necessary.

The elastomer composition may contain, in addition to the elastomer, an additive such as a plasticizer, an antioxidant, an age resistor, or a colorant; a dielectric filler, and the like.

The average thickness of the dielectric layer is preferably 10 $\mu$m to 1,000 $\mu$m and more preferably 30 $\mu$m to 200 $\mu$m, from the viewpoint of increasing capacitance C and thus promoting an increase in detection sensitivity and from the viewpoint of promoting an enhancement of followability for a measuring object.

It is preferable that the dielectric layer is deformable such that the length in the uniaxial direction increases by 30 % or more from an unelongated state. In a case where the dielectric layer having such characteristics is used in a state of being attached to a measuring object, the dielectric layer is suitable for changing the shape in conformity with the deformation of the measuring object.

Here, when it is said that a dielectric layer is deformable such that the length increases by 30 % or more, it is implied that the dielectric layer does not break even if the length is increased by 30 % (even if the elongation rate is 30 %) in a case where a load is applied to elongate the dielectric layer, and when the load is removed, the dielectric layer is restored to an original state (that is, being within an elastic deformation range).

The elongation rate at which the dielectric layer can be elongated in the uniaxial direction is more preferably 50 % or more, further preferably 100 % or more, and particularly preferably 200 % or more.

The elongation rate at which the dielectric layer can be elongated in the uniaxial direction can be controlled by design (materials, shapes, and the like) of the dielectric layer.

The relative permittivity at normal temperature of the dielectric layer is preferably 2 or higher, and more preferably 5 or higher. When the relative permittivity of the dielectric layer is less than 2, the capacitance C becomes smaller, and sufficient sensitivity as a sensor sheet may not be obtained.

Young's modulus of the dielectric layer is preferably 0.1 to 10 MPa. When this Young's modulus is less than 0.1 MPa, the dielectric layer becomes too soft, high quality processing is difficult, and sufficient measurement accuracy may not be obtained. On the other hand, when this Young's modulus is more than 10 MPa, the dielectric layer becomes too hard, and when the measuring object is about to change its shape, there is a risk that deformation thereof may be inhibited.

Hardness of the dielectric layer is preferably 0° to 30° as the hardness determined using a Type A durometer according to JIS K 6253 (JIS A hardness), or preferably 10° to 55° as the hardness determined using a Type C durometer according to JIS K 7321 (JIS C hardness).

If the dielectric layer is too soft, high quality processing is difficult, and sufficient measurement accuracy may not be secured. On the other hand, if the dielectric layer is too hard, when the measuring object is about to change its shape, there is a risk that deformation thereof may be inhibited.

In a case where the sensor sheet has a plurality of dielectric layers, it is not necessarily required that respective dielectric layers are composed of an elastomer composition having the same composition. Preferably, however, respective dielectric layers are composed of an elastomer composition having the same composition. This is because the respective dielectric layers show the same behavior when the sensor sheet is stretched and/or shrunk.

Electrode layer

The electrode layer is including an electroconductive composition containing an electroconductive material.

The respective electrode layers may be composed of electroconductive compositions having the same composition, or may be composed of electroconductive compositions having different composition. However, the respective electrode layers are preferably composed of electroconductive compositions having the same composition.

Examples of the electroconductive material include carbon nanotubes, graphene, carbon nanohorns, carbon fibers, electroconductive carbon black, graphite, metal nanowires, metal nanoparticles, and electroconductive polymers. These may be used singly, or two or more kinds thereof may be used in combination.

The electroconductive material is preferably carbon nanotubes. It is because carbon nanotubes are adequate for the formation of an electrode layer that changes its shape in conformity with the deformation of a dielectric layer.

Regarding the carbon nanotubes, known carbon nanotubes can be used. The carbon nanotubes may be single-walled carbon nanotubes (SWNT), or may be double-walled carbon nanotubes (DWNT) or multi-walled carbon nanotubes (MWNT) having three or more layers (in the present description, both are collectively referred to simply as multi-walled carbon nanotubes). Further, two or more kinds of carbon nanotubes having different number of layers may be used in combination.

The shape (average length, fiber diameter, and aspect ratio) of each of the carbon nanotubes is not particularly limited, and the shape may be appropriately selected after comprehensively considering the purpose of use of the sensor device, electric conductivity or durability required from the sensor sheet, and the treatment or cost for forming an electrode layer.

The average length of the carbon nanotubes is preferably 10 $\mu$m or more, and more preferably 50 $\mu$m or more. It is because an electrode layer formed using such carbon nanotubes having a long fiber length has excellent electric conductivity, and the electrode layer exhibits excellent characteristics such as that when the electrode layer changes its shape in conformity with the deformation of a dielectric layer (particularly when elongated), electrical resistance is hardly increased, and even if the electrode layer is subjected to repeated stretch, the electrode layer exhibits small variations in electrical resistance.

When the average length of the carbon nanotubes is less than 10 $\mu$m, electrical resistance may increase along with a deformation of the electrode layer, or the variation in the electrical resistance may increase when the electrode layer is subjected to repeated stretch. Particularly, in a case where the amount of deformation of the sensor sheet (dielectric layer) becomes large, such inappropriateness may easily occur.

A preferred upper limit of the average length of the carbon nanotubes is 1,000 $\mu$m. Currently, production and purchase of carbon nanotubes having an average length of more than 1,000 $\mu$m is practically infeasible. Furthermore, as will be described below, it is because in a case where an electrode layer is formed by applying a dispersion liquid of carbon nanotubes, since carbon nanotubes has unsatisfactory dispersibility, conductive paths are not easily formed, and consequently, there is a risk that the electrode layer may acquire insufficient electric conductivity.

The lower limit of the average length of the carbon nanotubes is further preferably 100 $\mu$m, and the upper limit is further preferably 600 $\mu$m. When the average length of the carbon nanotubes is within the above-described range, excellent characteristics such as that excellent electric conductivity is obtained, electrical resistance of the electrode layer is hardly increased at the time of elongation, and the variation in electrical resistance at the time of repeated stretch is small, can be more reliably secured to a higher level.

The fiber length of the carbon nanotubes may be determined by observing the carbon nanotubes with an electron microscope, and measuring the fiber length from the observation image.

The average length of the carbon nanotubes may be determined by, for example, calculating the average value based on the fiber lengths of the carbon nanotubes at ten sites randomly selected from observation images of the carbon nanotubes.

The average fiber diameter of the carbon nanotubes is not particularly limited; however, the average fiber diameter is preferably 0.5 nm to 30 nm.

When the fiber diameter is less than 0.5 nm, dispersion of the carbon nanotubes becomes poor, and as a result, conductive paths do not spread, and electric conductivity of the electrode layer may become insufficient. On the other hand, when the fiber diameter is more than 30 nm, the number of carbon nanotubes becomes smaller even at the same weight, and electric conductivity may become insufficient. The average fiber diameter of the carbon nanotubes is more preferably 5 to 20 nm.

Regarding the carbon nanotubes, multi-walled carbon nanotubes are preferred to single-walled carbon nanotubes.

If single-walled carbon nanotubes are used, even in a case where carbon nanotubes having an average length in the above-mentioned preferred range are used, electrical resistance may become high, electrical resistance may significantly increase at the time of elongation, or electrical resistance may be significantly vary at the time of repeated stretch.

The reason for this is speculated to be as follows. That is, since single-walled carbon nanotubes are usually synthesized as a mixture of metallic carbon nanotubes and semiconductive carbon nanotubes, it is speculated that the presence of these semiconductive carbon nanotubes is causative of increased electrical resistance, significant increase of electrical resistance at the time of elongation, or significant variation of electrical resistance at the time of repeated stretch.

If metallic carbon nanotubes are semiconductive carbon nanotubes are separated, and metallic single-walled carbon nanotubes having a long average length are used, there is a possibility that an electrode layer having electrical characteristics similar to those in the case of using multi-walled carbon nanotubes having a long average length may be formed.

However, separation of metallic carbon nanotubes and semiconductive carbon nanotubes is not easy (particularly, for carbon nanotubes having a long fiber length), and a complicated operation is needed for the separation of the two. Therefore, even from the viewpoint of the ease of operation at the time of forming an electrode layer and from the viewpoint of economic efficiency, multi-walled carbon nanotubes are preferred as the above-described carbon nanotubes.

It is preferable that the carbon nanotubes have a carbon purity of 99 % by weight or more. Carbon nanotubes may include catalytic metals, dispersants and the like during the production process therefor. In a case where carbon nanotubes containing components other than these carbon nanotubes (impurities) in large quantities are used, a decrease in electric conductivity or variation of electrical resistance may occur in the electrode layer.

The method for producing the carbon nanotubes is not particularly limited, and the carbon nanotubes may be produced by a conventionally known production method. However, it is preferable that carbon nanotubes produced by a substrate growth method are preferred.

A substrate growth method is a kind of CVD methods, and is a method for producing carbon nanotubes by supplying a carbon source to a metal catalyst applied on a substrate, and thereby growing carbon nanotubes. The substrate growth method is a production method suitable for producing carbon nanotubes having a relatively long fiber length and having an even fiber length. For this reason, this method is suitable for the carbon nanotubes that are used in the present invention.

In a case where the carbon nanotubes are produced by a substrate production method, the fiber length of the carbon nanotubes is substantially the same as the length of growth of the CNT forest. Therefore, in a case where the fiber length of carbon nanotubes is measured using an electron microscope, it is desirable to measure the length of growth of the CNT forest.

The electroconductive composition may also contain, for example, a binder component in addition to the electroconductive material such as carbon nanotubes.

The binder component functions as a binding material. Therefore, when the binder component is incorporated, the adhesion with the dielectric layer and the strength of the electrode layer itself can be enhanced. Furthermore, when an electrode layer is formed by a method as described below, scattering of the electroconductive material such as carbon nanotubes can be suppressed by incorporating the binder component, and therefore, safety at the time of forming an electrode layer can also be increased.

Examples of the binder component include butyl rubber, ethylene-propylene rubber, polyethylene, chlorosulfonated polyethylene, natural rubber, isoprene rubber, butadiene rubber, styrene-butadiene rubber, polystyrene, chloroprene rubber, nitrile rubber, polymethyl methacrylate, polyvinyl acetate, polyvinyl chloride, acrylic rubber, and a styrene-ethylene-butylene-styrene block copolymer (SEBS).

Furthermore, regarding the binder component, raw rubber (an unvulcanized natural rubber and an unvulcanized synthetic rubber) can also be used. When a material having relatively weak elasticity, such as raw rubber, is used, followability of an electrode layer to the deformation of a dielectric layer can also be increased.

It is particularly preferable that the binder component is of the same kind as the elastomer that constitutes the dielectric layer. It is because the adhesion between the dielectric layer and the electrode layer can be noticeably enhanced.

The electroconductive composition may further contain various additives in addition to the electroconductive material such as carbon nanotubes and the binder component. Examples of the additives include a dispersant for increasing dispersibility of the electroconductive material; a crosslinking agent, a vulcanization accelerator, a vulcanization aid for the binder component; an age resistor, a plasticizer, a softening agent, and a colorant.

In the sensor sheet described above, in a case where the electroconductive material is carbon nanotubes, the electrode layer may be substantially formed from carbon nanotubes only. In this case, also, sufficient adhesion between the electrode layer and the dielectric layer can be secured. In this case, carbon nanotubes and the dielectric layer strongly adhere to each other by van der Waals force or the like.

The content of the carbon nanotubes in the electrode layer is not particularly limited as long as the carbon nanotubes are included at a concentration at which electric conductivity is manifested. In a case where a binder component is incorporated, the content of the carbon nanotubes may vary depending on the kind of the binder component; however, the content is preferably 0.1 to 100 % by weight with respect to the total solid content of the electrode layer.

When the content of the carbon nanotubes is increased, electric conductivity of the electrode layer can be enhanced. Therefore, even if the electrode layer is made thin, required electric conductivity can be secured, and as a result, it becomes easier to make the electrode layer thinner or to secure flexibility of the electrode layer.

The average thickness of the electrode layer is preferably 0.1 to 10 $\mu$m. When the average thickness of the electrode layer is in the range described above, superior followability of the electrode layer to deformation of the dielectric layer can be manifested.

On the other hand, when the average thickness is less than 0.1 $\mu$m, there is a risk that electric conductivity may be insufficient, and the measurement accuracy as a sensor sheet may be decreased. On the other hand, when the average thickness is more than 10 $\mu$m, the sensor sheet becomes hard due to the reinforcement effect of the electroconductive material such as carbon nanotubes, stretchability of the sensor sheet is decreased. As a result, when the sensor sheet is attached directly to the living body surface or is attached indirectly to the living body surface to interpose a covering material therebetween, the deformation of the living body surface may be inhibited.

In the present invention, "the average thickness of the electrode layer" can be measured using, for example, a laser microscope (for example, manufactured by Keyence Corp., VK-9510). Specifically, an electrode layer formed on the top surface of a dielectric layer is scanned in the thickness direction in steps of 0.01 $\mu$m, a three-dimensional shape thereof is measured. Subsequently, the average height of a rectangular region having a size of 200 $\mu$m in length × 200 $\mu$m in width is measured from a region where the electrode layer is laminated on the dielectric layer and a region where the electrode layer is not laminated on the dielectric layer, and the level difference of the average heights may be designated as the average thickness of the electrode layer.

Cloth

The cloth has anisotropy in terms of stretchability. The anisotropy in terms of stretchability means that there is a significant difference in the degree of stretchability depending on directions. That is, the anisotropy in terms of stretchability means a characteristic in which the cloth is easy to stretch in one direction and is considerably difficult to stretch in a direction almost perpendicular to the one direction.

In the present description, the direction in which the cloth is easy to stretch is designated as an easily-stretchable direction and the direction in which the cloth is difficult to stretch is designated as a poorly-stretchable direction.

The cloth is not particularly limited as long as it has anisotropy in terms of stretchability, and may be woven fabric, knitted fabric, or nonwoven fabric.

The cloth has a ratio of modulus at 5 % in the poorly-stretchable direction to modulus at 5 % in the easily-stretchable direction ([M5 in the poorly-stretchable direction]/[M5 in the easily-stretchable direction], hereinafter, also referred to as "anisotropy M5") of preferably 5 or more and more preferably 10 or more.

When the cloth having a large anisotropy M5 is used, sensitivity and accuracy of the sensor sheet can be more improved.

The anisotropy M5 is more preferably 100 or more and further preferably 150 or more.

Further, the upper limit of the anisotropy M5 is not particularly limited, and a larger upper limit thereof is preferable.

On the other hand, even in the case of a cloth having stretchability, the cloth having a small difference between the stretchability in one direction and the stretchability in the direction almost perpendicular to the one direction, specifically, the cloth having a ratio of modulus at 5 % in the direction almost perpendicular to the one direction to modulus at 5 % in one direction of less than 2 does not corresponds to the stretchable anisotropic cloth in the present invention. Such a cloth is referred to as a stretchable isotropic cloth in the present description.

The modulus of the cloth is preferably larger than the modulus of the sensor body in the maximum use elongation rate of the sensor body.

In the capacitive sensor sheet of the present invention, the use elongation rate of the sensor body can be arbitrarily selected depending on the amount of deformation of the measuring object. For example, when the elongation rate of the sensor body becomes a maximum of 200 %, the maximum use elongation rate of the sensor body can be set to 200 %.

On the other hand, in a case where a measuring object is largely deformed beyond the predicted range of a user (measurer), for example, in a case where the sensor body having a maximum use elongation rate of 200 % is used, breakage may occur in the sensor body when the measuring object is largely deformed such that the sensor body is elongated beyond 200 %.

Herein, in the maximum use elongation rate of the sensor body (for example, 200 %), in a case where the cloth has a higher modulus than that of the sensor body (for example, in a case where modulus at 200 % of the cloth is larger than modulus at 200 % of the sensor body), the cloth functions as a stopper that suppresses the elongation of the sensor body beyond the predicted range, and thus it is possible to prevent the sensor body from being broken.

In the sensor sheet, it is preferred that the cloth is formed to cover the entirety of the detection portion when the sensor sheet is viewed in a planar view.

Accordingly, it is possible to reliably suppress the shrinkage of the sensor body in the direction (width direction) perpendicular to the elongation direction.

In the sensor sheet, as illustrated in FIGS. 2A, 2B, 3A and 3B, the cloth may be provided at both surfaces of the sensor body 10 or only at one surface of the sensor body.

In the sensor sheet, the cloth is integrally formed with the sensor body, for example, by using an adhesive.

Examples of the adhesive include an acrylic adhesive, a urethane-based adhesive, a rubber-based adhesive, and a silicone-based adhesive.

Here, the various adhesives may be solvent type adhesives, emulsion type adhesives, or hot melt type adhesives. The adhesive may be appropriately selected and used according to the use embodiment of the sensor device and the like. However, the adhesive needs to have flexibility that does not inhibit stretch of the dielectric layer.

Others

The sensor sheet may have a first conducting wire (top conducting wire), a second conducting wire (bottom conducting wire), and a third conducting wire, all of which are connected to the electrode layer, as shown in the examples illustrated in FIGS. 2A, 2B, 4A and 4B, as necessary.

These various wires may be any wires that do not inhibit shape change of the dielectric layer, and that maintain electric conductivity even if the dielectric layer is deformed. Specific examples of the various wires include, for example, conducting wires composed of an electroconductive composition similar to the electrode layer described above.

As shown in the examples illustrated in FIGS. 2A, 2B, 4A and 4B, a first connecting portion (top connecting portion), a second connecting portion (bottom connecting portion), and a third connecting portion for being each connected to external conducting wires may be formed at the end of each of the various conducting wires on the opposite side of the electrode layers, as necessary. Examples of these various connecting portions include connecting portions formed using copper foil or the like.

As shown in the examples illustrated in FIGS. 2A, 2B, 4A and 4B, the sensor body may have a protective layer laminated at the outermost layer on the top side and/or the bottom side, as necessary. When the protective layer is provided, electroconductive regions (such as the electrode layers) of the sensor sheet can be protected or the sensor sheet can be increased in strength and endurance.

The material of the protective layer is not particularly limited, and the material may be appropriately selected in accordance with properties required for the layer. As a specific example of the material, for example, an elastomer composition or the like similar to the material of the dielectric layer is mentioned.

As described above, the sensor sheet may have an adhesive layer formed at the outermost layer on the bottom side of the sensor sheet. Thereby, the sensor sheet can be attached to a measuring object such as a living body surface through this adhesive layer.

The adhesive layer is not particularly limited, and examples thereof include layers formed from an acrylic adhesive, a urethane-based adhesive, a rubber-based adhesive, a silicone-based adhesive, and the like.

Here, the various adhesives may be solvent type adhesives, emulsion type adhesives, or hot melt type adhesives. The adhesive may be appropriately selected and used according to the use embodiment of the capacitive sensor and the like. However, the adhesive layer needs to have flexibility that does not inhibit stretch/shrinkage of the dielectric layer.

As in an example illustrated in FIGS. 3A and 3B, the sensor sheet may include a non-stretchable member to cover portions (a conducting wire and a connecting portion) other than the detection portion of the sensor body. As described above, this is because measurement sensitivity is improved.

The non-stretchable member is not particularly limited as long as it is formed from a non-stretchable material, and examples thereof include a non-stretchable cloth and a non-stretchable resin sheet.

As in an example illustrated in FIGS. 3A and 3B, the sensor sheet may include a handle as necessary.

The material and shape of the handle are not particularly limited as long as they do not impair the performance of the sensor body, and may be appropriately selected in consideration of use embodiments.

When the sensor sheet is subjected to repetition of 1,000 cycles of stretch, for which a cycle of elongating the sensor sheet 100 % in a uniaxial direction from an unelongated state and then returning the sensor sheet to an unelongated state is defined as one cycle, it is preferable that the change ratio of electrical resistance of the electrode layer at the time of 100 % elongation of the 1,000-th cycle with respect to the electrical resistance of the electrode layer at the time of 100 % elongation of the second cycle, ({absolute value of [electrical resistance value at the time of 100 % elongation of the 1,000-th cycle] - [electrical resistance value at the time of 100 % elongation of the second cycle]} / [electrical resistance value at the time of 100 % elongation of the second cycle] $\times$ 100), is small. Specifically, the change ratio is preferably 10 % or less, and more preferably 5 % or less.

Here, the reason why the electrical resistance of the electrode layer after the second cycle, not the first cycle, is applied as the object of evaluation, is that at the time of the first elongation (first cycle) of elongating the sensor sheet from an unelongated state, the behavior of the electrode layer (mode of fluctuation in electrical resistance) at the time of stretch after the second elongation (second cycle). Regarding a cause for this, it is speculated that after a sensor sheet is produced, the state of carbon nanotubes or the like that constitute the electrode layer is stabilized for the first time by elongating the sensor sheet one time.

The sensor sheet can be produced, for example, through the following steps. Here, a case where an electrode layer is formed by using an electroconductive composition containing carbon nanotubes will be described as an example.

The sensor sheet is produced through

(1) a step of producing a dielectric layer composed of an elastomer composition (step (1)),
(2) a step of applying a composition containing carbon nanotubes and a dispersing medium to the dielectric layer to form an electrode layer (step (2)), and
(3) a step of integrally forming a sensor body produced through the steps (1) and (2) with a cloth.

Step (1)

In this step, the dielectric layer composed of an elastomer composition is produced.

First, a raw material composition is produced by mixing an elastomer (or a raw material thereof) as a raw material composition, optionally with an additive such as a chain extending agent, a crosslinking agent, a vulcanization accelerator, a catalyst, a dielectric filler, a plasticizer, an antioxidant, an age resistor, or a colorant. Next, a dielectric layer is produced by molding this raw material composition. Regarding the method for molding the raw material composition, a conventionally known technique can be employed.

Specifically, for example, in a case where a dielectric layer containing urethane elastomer is molded, a method described below or the like can be used.

First, a polyol component, a plasticizer, and an antioxidant are weighed, and these components are mixed with stirring for a certain time under heating and reduced pressure to produce a mixed liquid. Next, this mixed liquid is weighed, the temperature is adjusted, subsequently a catalyst is added thereto, and the mixture is stirred with an agitator or the like. Subsequently, a predetermined amount of an isocyanate component is added thereto, and the mixture is stirred with an agitator or the like.

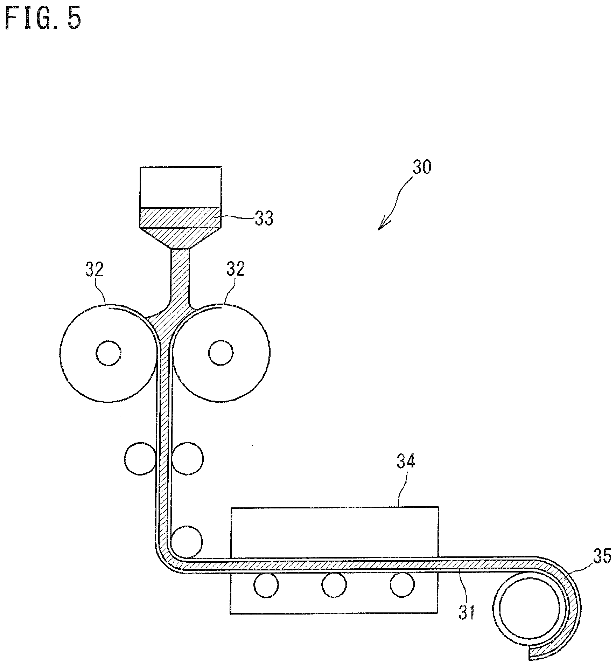

Subsequently, the mixed liquid is instantly poured into the forming apparatus illustrated in FIG. 5, the mixed liquid is produced into a sandwich form with protective films, and the mixed liquid is crosslinked and cured while being conveyed. Thus, a protective film-attached sheet having a predetermined thickness is obtained. Subsequently, the sheet is crosslinked in a furnace after a certain time, and finally, the sheet is cut into a predetermined shape. Thereby, a dielectric layer can be produced.

FIG. 5 is a schematic diagram for explaining an example of a forming apparatus used for the production of a dielectric layer. In a forming apparatus 30 illustrated in FIG. 5, a raw material composition 33 is caused to flow into a gap between protective films 31 formed from polyethylene terephthalate (PET), which are continuously sent from a pair of rolls 32, 32 that are disposed apart from each other, and while a curing reaction (crosslinking reaction) is carried out in a state in which the raw material composition 33 is maintained in the gap, the assembly is introduced into a heating apparatus 34. The raw material composition 33 is thermally cured in a state in which the raw material composition 33 is maintained between the pair of protective films 31, and thus a sheet-like dielectric layer 35 is formed.

The dielectric layer may be produced after the raw material composition is prepared, using a general-purpose film forming apparatus or a film forming method, such as various coating apparatuses, bar coating, or a doctor blade.

Step (2)

In this step, a composition containing carbon nanotubes and a dispersing medium (carbon nanotube dispersion liquid) is applied and then the dispersing medium is removed in a drying process, thereby forming an electrode layer integrated with the dielectric layer.

Specifically, first, carbon nanotubes are added to a dispersing medium. At this time, if necessary, the above-mentioned other components such as a binder component (or a raw material for a binder component), or a dispersant may be further added thereto.

Next, various components including carbon nanotubes are dispersed (or dissolved) in the dispersing medium in a wet dispersing machine, and thereby an application liquid (carbon nanotube dispersion liquid) is prepared. Here, for example, dispersing may be performed using an existing dispersing machine such as an ultrasonic dispersing machine, a jet mill, or a bead mill.

Examples of the dispersing medium include toluene, methyl isobutyl ketone (MIBK), alcohols, and water. These dispersing media may be used singly, or two or more kinds thereof may be used in combination.

Regarding the application liquid, the concentration of the carbon nanotubes is preferably 0.01 to 10 % by weight. When the concentration is less than 0.01 % by weight, the concentration of carbon nanotubes is too low, and it may be necessary to repeatedly apply the application liquid. On the other hand, when the concentration is more than 10 % by weight, the viscosity of the application liquid becomes too high, dispersibility of the carbon nanotubes is decreased as a result of reaggregation, and it may be difficult to form a uniform electrode layer.

Subsequently, the application liquid is applied to a predetermined position on the top surface of the dielectric layer by a method such as spray coating, and then dried. At this time, the position, at which the electrode layer is not formed, on the top surface of the dielectric layer is masked, and then the application liquid may be applied.

The drying conditions for the application liquid are not particularly limited, and the drying conditions may be appropriately selected according to the type of the dispersing medium, the composition of the elastomer composition, or the like.

Furthermore, the method for applying the application liquid is not intended to be limited to spray coating, and in addition to that, for example, a screen printing method, an inkjet printing method, and the like can also be employed.

After the dielectric layer and the electrode layer have been formed through the above steps (1) and (2), the conducting wires connected to the electrode layers and the connecting portions are formed as necessary.

The conducting wire to be connected to the electrode layer is formed by the same method as used to form the electrode layer such that the carbon nanotube dispersion liquid (application liquid) is applied to a predetermined position and dried. Furthermore, the conducting wire may be simultaneously formed with the electrode layer.

The connecting portion may be formed, for example, by attaching copper foil or the like to the predetermined end of the conducting wire.

When a sensor body having a structure as illustrated in FIGS. 4A and 4B is produced, first, two dielectric layers each composed of an elastomer composition are produced by the method in the step (1). Next, electrode layers are formed on both surfaces of one of the dielectric layers by the method in the step (2), and an electrode layer is formed on one surface of the other dielectric layer. Thereafter, the two dielectric layers, in each of which the electrode layer(s) is/are formed, are attached to each other not to put any one of the electrode layers onto the other electrode layers. At this time, as necessary, conducting wires and connecting portions that are each connected to the electrode layers.

Thereafter, a protective layer may be further formed in the outermost layer on the top side and/or the bottom side.

For the formation of the protective layer, it is advisable, for example, to use the same method as used in the step (1) to produce a sheet-like product composed of an elastomer composition, cut the product into a predetermined size, and then laminate the piece(s) on the electrode-layer-formed workpiece.

When a sensor sheet including a protective layer is produced, the sensor sheet may be produced by forming a bottom protective layer as a start of the production, and then laminating, thereon, constituent members (a second electrode layer, a first dielectric layer, a first electrode layer, (a second dielectric layer and a third electrode layer), and a top protective layer) successively.

Through such steps, the sensor body can be produced.

Step (3)

In this step, a cloth is attached to the sensor body.

First, a stretchable anisotropic cloth, has a predetermined direction, and is cut into a predetermined size is prepared.

Next, an adhesive layer is formed on one surface of the cloth, the cloth is attached to the sensor body through the adhesive layer such that the easily-stretchable direction of the cloth is consistent with the elongation direction of the sensor body. Incidentally, the order of cutting of the cloth and forming of the adhesive layer may be reversed.

In this step, the cloth may be attached to both surfaces of the sensor body or may be attached only to one surface of the sensor body.

The cloth is attached so as to cover at least the detection portion of the sensor body in a planar view.

Further, when the cloth is attached to both surfaces of the sensor body, the sensor body may be buried between two clothes and the entire outer peripheries of the clothes may be attached to each other.

In this step, as necessary, an unshrinkable member or a handle may be attached.

Through such steps, the sensor sheet of the present invention can be produced.

The sensor body illustrated in FIGS. 2A, 2B, 4A and 4B has a single detection portion. However, the number of the detection portions of the sensor body is not limited to one. Thus, the sensor body may have a plurality of detection portions.

A specific example of the sensor body having a plurality of detection portions is a sensor body in which an electrode layer in the form of rectangles as plural rows is formed on each of the top surface and the bottom surface of the dielectric layer and the rows of the top electrode layer are arranged to be perpendicular to the rows of the bottom electrode layer in a planar view. In such a sensor body, a plurality of parts where the top electrode layer and the bottom electrode layer face each other with the dielectric layer interposed therebetween are designated as detection portions, and the detection portions are arranged in a lattice form.

Measuring Instrument

The measuring instrument is electrically connected to the sensor sheet (the sensor body). The measuring instrument has a function of measuring capacitance C of the detection portion, which changes with deformation of the dielectric layer. The method for measuring the capacitance C may be a conventionally known method. Thus, the measuring instrument has a capacitance measuring circuit, an arithmetic circuit, an amplifier circuit, a power supply circuit, and the like that are needed for the measurement of capacitance.

The method (circuit) for measuring the capacitance C is, for example, a CV conversion circuit (such as an LCR meter) utilizing an auto-balancing bridge circuit, a CV conversion circuit utilizing an inverting amplifier circuit, a CV conversion circuit utilizing a half-wave voltage doubler rectifier circuit, a CF oscillation circuit utilizing a Schmitt trigger oscillator circuit, or a method in which a Schmitt trigger oscillator circuit is combined with an F/V conversion circuit.

It is preferable in the sensor element of the present invention that the sensor sheet is connected to measuring instrument in a manner described below.

(1-1) Case in which the sensor body is a sensor body as illustrated in FIGS. 4A and 4B, which has two dielectric layers (first and second dielectric layers) and respective electrode layers (first to third electrode layers) on both surfaces of each of the dielectric layers; and the measuring instrument is a measuring instrument utilizing a CF oscillation circuit (such as a Schmitt trigger oscillator circuit) that is oscillated by the capacitance C and the resistance R of the detection portion, thereby measuring a change in the capacitance.

In this case, it is preferred to connect the first electrode layer to the oscillation block (detecting block) and further earth the second electrode layer and the third electrode layer (connect the electrodes to the GND side).

Such a connection of the sensor body to the measuring instrument makes it possible to exclude noises even when either the top side or the bottom side of the sensor body is connected to a measuring object such as a living body to be made close to and contactable with the measuring object. As a result, a change in the capacitance is more precisely measurable.

(1-2) Case in which the sensor body is a sensor body as illustrated in FIGS. 4A and 4B, which has two dielectric layers and respective electrode layers on both surfaces of each of the dielectric layers; and the measuring instrument is a measuring instrument utilizing a CV conversion circuit, such as a half-wave voltage doubler rectifier circuit, an inverting amplifier circuit, or an auto-balancing bridge circuit, for passing alternating signals generated in a different block (for example, an AC applying device) through the sensor body and then generating a voltage change by measuring or using a change in the alternating impedance of the sensor body by a change in the capacitance of the sensor body.

In this case, it is preferred to connect the first electrode layer to the detecting block, and connect the second electrode layer and the third electrode layer to the block for generating the alternating signals.

Such a connection of the sensor body to the measuring instrument makes it possible to exclude noises even when either the top side or the bottom side of the sensor body is connected to a measuring object such as a living body to be made close to and contactable with the measuring object. As a result, a change in the capacitance is more precisely measurable.

(2-1) Case in which the sensor body is a sensor body as illustrated in FIGS. 2A and 2B, which has a single dielectric layer and respective electrode layers (top electrode layer and bottom electrode layer) on both surfaces of this dielectric layer; and the measuring instrument is a measuring instrument utilizing a CF conversion circuit such as a Schmitt trigger oscillator circuit

In this case, it is preferred to connect the top electrode layer to an oscillation block (detecting block) in the measuring instrument, earth the bottom electrode layer (connect this layer onto the GND side), and further attach the sensor body to a measuring object such as a living body to make the bottom surface side of the sensor body close to and contactable with the measuring object.

By attaching the sensor body to the living body or the like in this direction and connecting the sensor body to the measuring instrument as described above, the effect of noises can be excluded. As a result, a change in the capacitance is more precisely measurable.

(2-2) Case in which the sensor body is a sensor body as illustrated in FIGS. 2A and 2B, which has a single dielectric layer and respective electrode layers (top electrode layer and bottom electrode layer) on both surfaces of this dielectric layer; and the measuring instrument is a measuring instrument utilizing a CV conversion circuit such as a half-wave voltage doubler rectifier circuit, an inverting amplifier circuit, or an auto-balancing bridge circuit.

In this case, it is preferred to connect the top electrode layer to a detecting block in the measuring instrument, connect the bottom electrode layer to the block for generating alternating signals, and further attach the sensor body to a measuring object such as a living body to make the bottom surface side of the sensor body close to and contactable with the measuring object.

By attaching the sensor body to the living body or the like in this direction and connecting the sensor body to the measuring instrument as described above, the effect of noises can be excluded. As a result, a change in the capacitance is more precisely measurable.

Display Device

The sensor device of the present invention may include a display device as in the case of the example shown in FIG. 1. Thereby, a user of the sensor device can check the information on the change of capacitance C by the deformation of the measuring object in real time. The display device includes a monitor, an arithmetic circuit, an amplifier circuit, a power supply circuit and the like, which are needed for that purpose.

Furthermore, in order to store the measurement results for capacitance C as in the case of the example shown in FIG. 1, the display device may also include a memory unit such as a RAM, a ROM, or a HDD.

For example, when the sensor device of the present invention is used for a person who is in sports training or rehabilitation training, the display device makes it possible to verify, after the training, information pieces based on a change in the capacitance C regrading, for example, living body motion information pieces. For this reason, the person can check the achievement level of the training. Thus, this matter can encourage the person. Moreover, the achievement level of the training can be checked to make use of the information pieces to prepare a new training-menu.

The above-mentioned measuring instrument may have the memory unit. As the display device, a terminal instrument such as a personal computer, a smartphone, or a tablet may be used.

Furthermore, in regard to the sensor device 1 illustrated in FIG. 1, connection between a measuring instrument 3 and the display device 4 is wired; however, the connection thereof in the sensor device of the present invention is not necessarily required to be wired, and wireless connection is also acceptable. According to the use embodiment of the sensor device, it may be easier to use a sensor in which the measuring instrument and the display device are physically separated.

EXAMPLES

Hereinafter, the present invention will be more specifically described by way of Examples; however, the present invention is not intended to be limited to the following Examples.

Production of sensor body

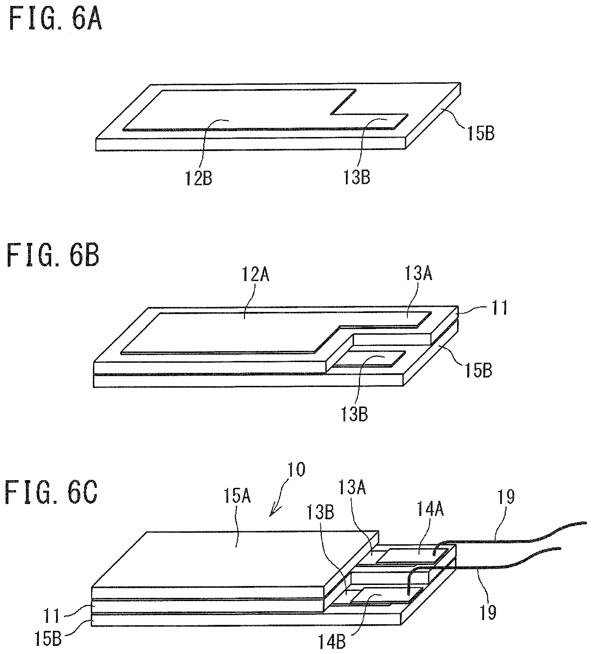

FIGS. 6A to 6C are perspective views for explaining production processes for a sensor body in Example. Here, a sensor body illustrated in FIGS. 2A and 2B was produced.

(1) Production of dielectric layer

To 100 parts by mass of a polyol (PANDEX GCB-41, manufactured by DIC Corp.), 40 parts by weight of a plasticizer (dioctyl sulfonate) and 17.62 parts by weight of an isocyanate (PANDEX GCA-11, manufactured by DIC Corp.) were added, and the mixture was mixed with stirring for 90 seconds using an agitator. Thus, a raw material composition for a dielectric layer was prepared. Next, the raw material composition was poured into a forming apparatus 30 such as illustrated in FIG. 5, and the raw material composition was produced into a sandwich form with protective films 31.

While the assembly was conveyed, the raw material composition was cured by crosslinking under the conditions of a furnace internal temperature of 70 °C and a furnace retention time of 30 minutes, and thus a rolled sheet having protective films attached thereto and having a predetermined thickness was obtained. Subsequently, the rolled sheet was kept for 12 hours in a furnace that had been adjusted to 70 °C and then was crosslinked. Thus, a sheet formed from polyether-based urethane elastomer was produced. The urethane sheet thus obtained was cut into a size of 14 mm × 80 mm × 100 $\mu$m in thickness. Furthermore, one place of a corner part was cut out into a size of 7 mm × 20 mm × 100 $\mu$m in thickness, and thus a dielectric layer was produced.

For the dielectric layer thus produced, the elongation at break (%) and the relative permittivity were measured. As a result, the elongation at break (%) was 505 %, and the relative permittivity was 5.8.

Here, the elongation at break was measured according to JIS K 6251. Regarding the relative permittivity, a dielectric layer was interposed between electrodes having a diameter of 20 mm$\phi$, and capacitance was measured at a measurement frequency of 1 kHz using a LCR HITESTER (manufactured by Hioki E.E. Corp., 3522-50). Thus, the relative permittivity was calculated from the electrode area and the thickness of the measurement sample.

(2) Production of electrode layer material

Thirty mg of highly oriented carbon nanotubes manufactured by Taiyo Nippon Sanso Corp. (number of layers: 4 to 12 layers, fiber diameter: 10 to 20 nm, fiber length: 150 $\mu$m to 300 $\mu$m, and carbon purity: 99.5 %), which were multi-walled carbon nanotubes produced by a substrate growth method, were added to 30 g of isopropyl alcohol (IPA), and the mixture was subjected to a wet dispersing treatment using a jet mill (NANO JET PUL JN10-SP003, manufactured by JOKOH CO., LTD.). The dispersion was diluted to 2 times, and thus a carbon nanotube dispersion liquid having a concentration of 0.05 % by weight was obtained.

(3) Production of protective layer

The same method as in (1) Production of dielectric layer described above was used to produce a bottom protective layer of 14 mm × 80 mm × 50 $\mu$m in thickness and a top protective layer of 14 mm × 60 mm × 50 $\mu$m in thickness which were each composed of the polyether-based urethane elastomer.

(4) Production of sensor body

A mask produced by forming an opening having a predetermined shape in a release-treated PET film (not shown in the diagram), was attached to one surface (top surface) of the bottom protective layer 15B produced in the process of (3).

In the mask, an opening corresponding to the position of the bottom electrode layer and the bottom conducting wire is formed, and the size of the opening is such that the portion corresponding to the bottom electrode layer has a size of 10 mm in width × 50 mm in length, and the portion corresponding to the bottom conducting wire has a size of 5 mm in width × 20 mm in length.

Next, the carbon nanotube dispersion liquid produced in the process of (2) was applied thereon from a distance of 10 cm using an airbrush such that the amount of the dispersion liquid applied per unit area (cm$^2$) was 0.223 g. Subsequently, the dispersion liquid was dried for 10 minutes at 100°C to thereby form a bottom electrode layer 12B and a bottom conducting wire 13B. Subsequently, the mask was detached (see FIG. 6A).

Next, the dielectric layer 11 produced in the process of (1) was laminated on the bottom protective layer 15B by superimposing the dielectric layer 11 thereon so as to cover the entirety of the bottom electrode layer 12B and a portion of the bottom conducting wire 13B.

Furthermore, on the top side of the dielectric layer 11, a top electrode layer 12A and a top conducting wire 13A were formed using the same method as that used for the formation of the bottom electrode layer 12B and the bottom conducting wire 13B (see FIG. 6B).

Next, on the top side of the dielectric layer 11 where the top electrode layer 12A and the top conducting wire 13A were formed, the top protective layer 15A produced in the process of (3) was laminated with a laminator so as to cover the entirety of the top electrode layer 12A and a portion of the top conducting wire 13A.

Furthermore, copper foil was attached to the respective ends of the top conducting wire 13A and the bottom conducting wire 13B, and these were designated as a top connecting portion 14A and a bottom connecting portion 14B, respectively. Next, lead wires 19 that served as external conducting wires were fixed by soldering to the top connecting portion 14A and the bottom connecting portion 14B to obtain a sensor body 10 (see FIG. 6C).

Preparation of cloth

As a cloth, the following clothes were prepared.

(a) Stretchable anisotropic cloth A: Super Stretch II (purchased from SANTOKU CORPORATION, quality: nylon 90 %, polyurethane 10 %, thickness: 600 $\mu$m)
(b) Stretchable anisotropic cloth B: PIP KINESIOLOGY (registered trademark) manufactured by PIP CO., LTD., thickness: 600 $\mu$m
(c) Stretchable isotropic cloth A: 2-WAY Tricot (purchased from Yuzawaya Shoji Co., Ltd., quality: nylon 85 %, polyurethane 15 %, thickness: 700 $\mu$m)
(d) Stretchable isotropic cloth B: Lycra Mat (purchased from Crystal Clover K.K., quality: nylon 83 %, polyurethane 17 %, thickness: 600 $\mu$m)
(e) Stretchable isotropic cloth C: product under development by TORAY INDUSTRIES, INC. (quality: polyester 95 %, polyurethane 5 %, thickness: 350 $\mu$m)

Production of adhesive layer

A machine AWATORI RENTARO (model No.: ARE-310, manufactured by Thinky Corp.) was used to mix 50 parts by weight of an adhesive (SK-Dyne 1720, manufactured by Soken Chemical & Engineering Co., Ltd.) with 50 parts by weight of methyl ethyl ketone (MEK) and 2 parts by mass of a curing agent (L-45, manufactured by Soken Chemical & Engineering Co., Ltd.) (at 2,000 rpm for 120 seconds), and degas the resultant (at 2,000 rpm for 120 seconds) to obtain a mixture. Next, an applicator was used to form the obtained mixture into a film having a wet film thickness of 100 $\mu$m onto a PET film (50E-0010KF, manufactured by Fujimori Kogyo Co., Ltd.), the top surface of which had been subjected to releasing treatment. Thereafter, a blower type oven was used to cure the film at 100°C for 30 minutes to produce an adhesive layer having a thickness of 30 $\mu$m after the curing.

Example 1

A sensor sheet in which the sensor body is integrally formed with the cloth was produced according to a method described below.

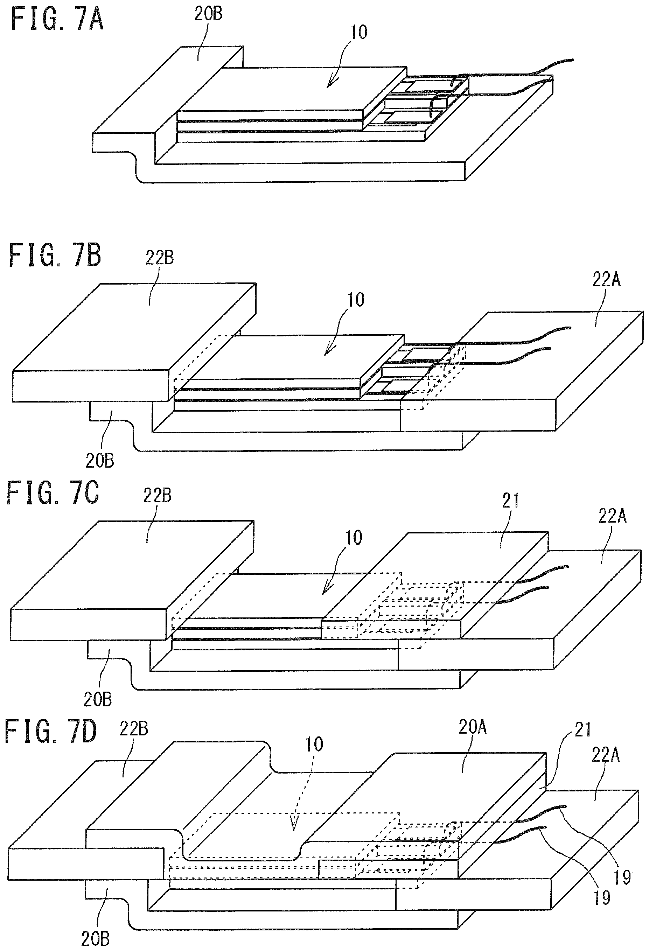

FIGS. 7A to 7D are perspective views for explaining a method for producing a sensor sheet in Example.

(1) The adhesive layer produced by the above-described method was transferred to one surface of the stretchable anisotropic cloth A.
Thereafter, the stretchable anisotropic cloth A was cut into a size of 115 mm in width $\times$ 30 mm in height. At this time, cutting was carried out such that the width became the easily-stretchable direction and the height became the poorly-stretchable direction.
(2) The cut stretchable anisotropic cloth 20B was attached to the bottom surface side of the sensor body 10 produced by the above-described method. At this time, the stretchable anisotropic cloth 20B was attached such that the end on the opposite side to the side where the conducting wires (the top conducting wire 13A and the bottom conducting wire 13B) of the sensor body 10 was positioned at the position distant from one end in the longitudinal direction (the width direction) of the stretchable anisotropic cloth 20B by 15 mm (see FIG. 7A).
(3) Next, nylon woven belts 22A and 22B as handles for fixing a sensor sheet to a tensile tester at the time of a test to be described later were attached to both ends of the stretchable anisotropic cloth 20B (see FIG. 7B). At this time, attachment of the woven belts 22A and 22B was performed using a double-sided tape (No. 777 manufactured by TERAOKA SEISAKUSHO CO., LTD.). Further, the woven belt 22B was attached such that the end thereof was also disposed at a portion of the upper surface of the sensor body 10 (at a position not overlapping with the detection portion in a planar view).

(4) Next, in order to prevent stretch/shrinkage of the dielectric layer other than the detection portion in the sensor body 10, a non-stretchable cloth (Decine Sebuse manufactured by WATANABE MFG. CO., LTD., polyester 100 % woven cloth, 45 mm in width × 30 mm in height) 21 was attached through a double-sided tape (No. 777 manufactured by TERAOKA SEISAKUSHO CO., LTD.) so as to cover the conducting wires (the top conducting wire 13A and the bottom conducting wire 13B) and the connecting portions (the top connecting portion 14A and the bottom connecting portion 14B) (see FIG. 7C).

(5) Thereafter, the stretchable anisotropic cloth 20A, which had been cut in (1), was also attached to the top surface side of the sensor body, thereby completing a sensor sheet 2 (see FIG. 7D).

Example 2

A sensor sheet was produced in the same manner as in Example 1, except that a stretchable anisotropic cloth B was used instead of the stretchable anisotropic cloth A.

However, the stretchable anisotropic cloth B was a product having an adhesive layer on one surface thereof, and the stretchable anisotropic cloth B was attached through the adhesive layer of the stretchable anisotropic cloth B without transferring the adhesive layer.

Comparative Example 1

A sensor sheet was produced in the same manner as in Example 1, except that a stretchable isotropic cloth A was used instead of the stretchable anisotropic cloth A.

Comparative Example 2

A sensor sheet was produced in the same manner as in Example 1, except that a stretchable isotropic cloth B was used instead of the stretchable anisotropic cloth A.

Comparative Example 3

A sensor sheet was produced in the same manner as in Example 1, except that a stretchable isotropic cloth C was used instead of the stretchable anisotropic cloth A.

Comparative Example 4

The same non-stretchable cloth as in Example 1 was attached through a double-sided tape (No. 777 manufactured by TERAOKA SEISAKUSHO CO., LTD.) onto the conducting wires (the top conducting wire 13A and the bottom conducting wire 13B) and the connecting portions (the top connecting portion 14A and the bottom connecting portion 14B) of the sensor body 10 produced by the above-described method and to ends that are on the opposite side (the left side in FIG. 6C) to the side, at which the conducting wires and the connecting portions are provided, of the upper surface of the top protective layer 15A and are positions not overlapping with detection portions. Then, the resultant product was used as a sensor sheet (measurement sample) for evaluation to be described below.

Evaluation

The following evaluation on the sensor sheets according to Examples and Comparative Examples were carried out. The results thereof are shown in Table 1.

(1) Tensile characteristics of cloth

Regarding the tensile characteristics of the clothes used in Examples 1 and 2 and Comparative Examples 1 to 3, "modulus at 5 % (M5)", "modulus at 10 % (M10)", "modulus at 50 % (M50)", "modulus at 100 % (M100)", "modulus at 200 % (M200)", "Elongation at break", "Load at break", and "Constant load elongation" were measured and "Anisotropy M5" and "Anisotropy M50" were calculated.

Herein, for the measurement of "Constant load elongation", the elongation rate at 600 N/m (about five times M200 of Comparative Example 4) was measured. Further, "Anisotropy M5" and "Anisotropy M50" were calculated on the basis of the measurement values of "M5" and "M50" in each of the width and the height.

Specifically, the cloth was cut into a size of 35 mm in width × 100 mm in length to be used as a test piece. At this time, three pieces of perpendicularly intersecting samples in the length direction each were prepared (for example, in the case of preparing a test piece of the stretchable anisotropic cloth A, three samples having an easily-stretchable direction (width direction) as the length direction and three samples having a poorly-stretchable direction (height direction) as the length direction were prepared).

Then, 25 mm of both ends in the length direction of each sample were pinched with chucks, measurement was carried out at a tension rate of 500 mm/min using a universal tensile/compression tester (Instron Model 1175), and then "anisotropy M5" and "anisotropy M50" were calculated.

The average values are shown as the results in Table 1.

(2) Characteristic of sensor sheet

The sensor sheets according to Examples and Comparative Examples were attached to a universal tensile/compression tester (Instron Model 1175), the sensor sheets were pulled out at an interval of 5 mm (at an interval of elongation rate 10 %), and then the capacitance was measured at a measurement frequency of 5 kHz using LCR HITESTER (manufactured by Hioki E.E. Corp., 3522-50) in a state where the sensor sheets were stood still.

(2-1) Sensitivity evaluation

In the above-described measurement, sensitivity (pF/%) of the sensor sheet was calculated by the following Calculation Formula (2) on the basis of the capacitance at the time of unelongation (0 % elongation) and the capacitance at the time of 40 % elongation.

$$\text{Sensitivity (pF/\%)}$$
$$= [\text{amount of change of capacitance } \Delta C_{0\% \to 40\%}]/40 \% \qquad ...(2).$$

(2-2) Hysteresis evaluation

The sensor sheet was attached to the tensile tester and elongated up to a predetermined elongation rate (40 % in Example 2 and 80 % in other Examples and Comparative Example) from unelongation (0 %), and then the stretch/shrinkage operation of returning the sensor sheet to the unelongated state was performed for three reciprocations. Thereafter, hysteresis (%) of the sensor sheet was calculated by the following Calculation Formula (3) on the basis of the capacitance at the time of 10 % elongation in the path of the forward operation at the first reciprocation (capacitance (the first path of the forward operation, 10 %)) and the capacitance at the time of 10 % elongation in the path of the forward operation at the second reciprocation (capacitance (the second path of the forward operation, 10 %)).

$$\text{Hysteresis (\%)}$$
$$= [\text{capacitance (the second path of the forward operation, 10 \%)}]$$
$$/[\text{capacitance (the first path of the forward operation, 10 \%)}] - 1) \times 100$$
$$...(3).$$

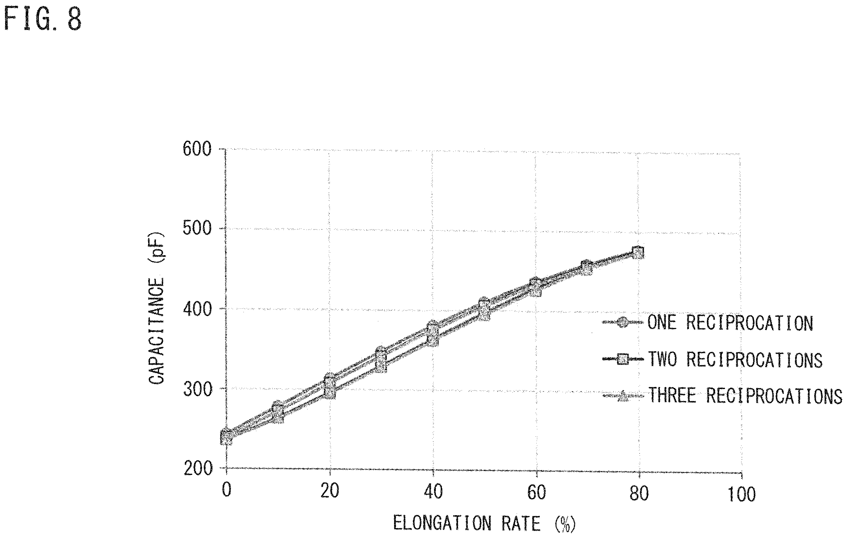

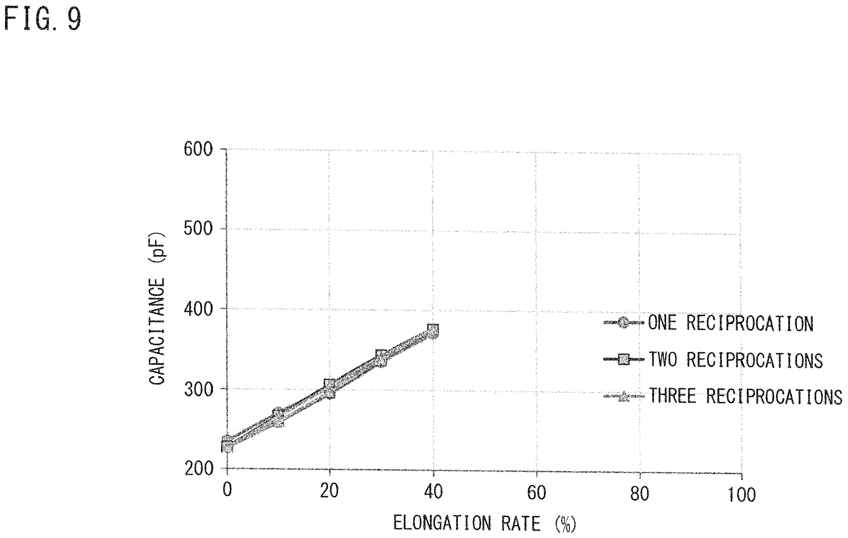

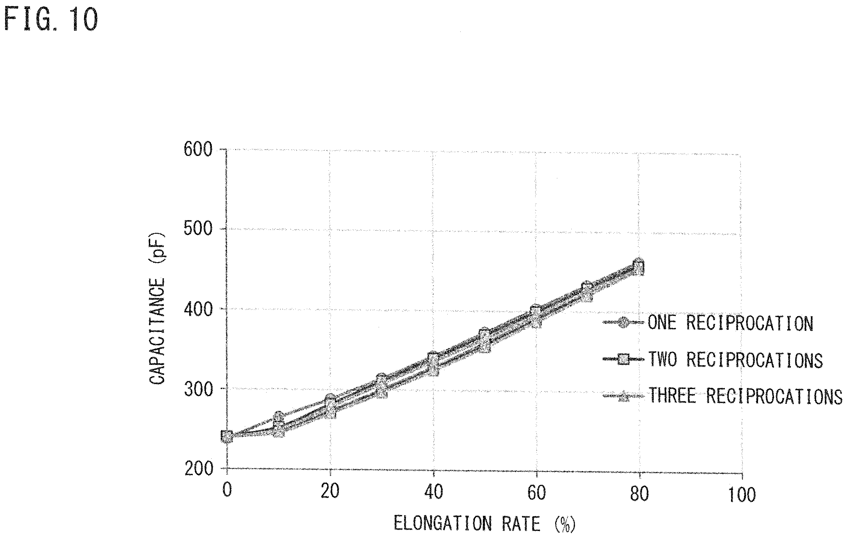

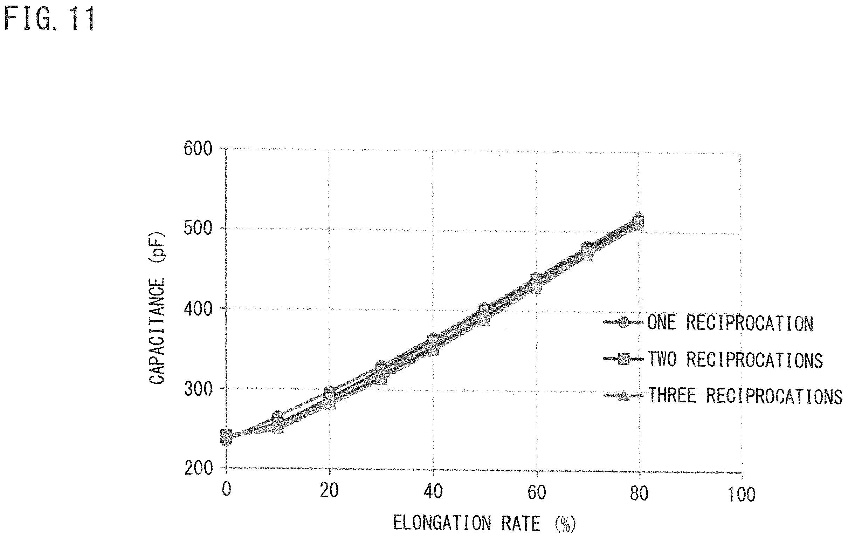

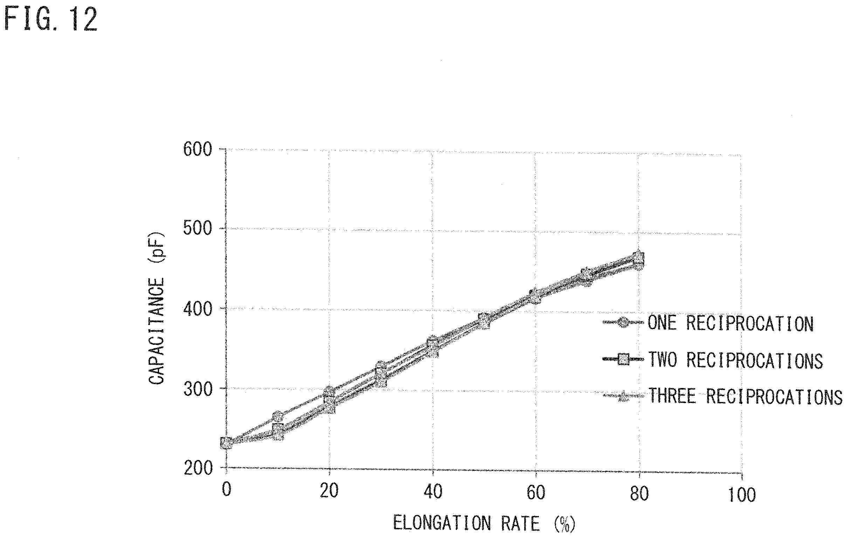

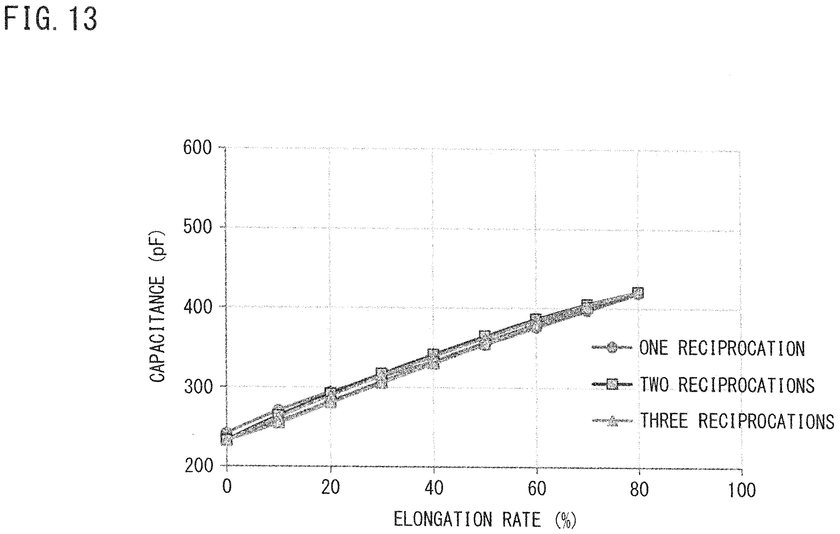

Further, FIGS. 8 to 13 show the graphs of measurement results of capacitances at the time of a reciprocatory elongation operation.

Table 1

| | | | | Tensile characteristics of cloth ※1 | | | | | | | | Characteristic of sensor sheet | | | |
|---|---|---|---|---|---|---|---|---|---|---|---|---|---|---|---|---|
| | | Cloth | | | | | | | | | | | | | | |
| | Type | Direction | Thickness | M5 | M10 | M50 | M100 | M200 | Elongation at break | Load at break | Constant load elongation | Anisotropy | Anisotropy | Sensitivity | Hysteresis |
| | | | (μm) | (N/m) | (N/m) | (N/m) | (N/m) | (N/m) | (%) | (N/m) | (%) | M5 | M50 | (pF/%) | (%) |
| Example 1 | Stretchable anisotropic cloth A | Width | 600 | 14.2 | 21.5 | 77.4 | 174.9 | 760.4 | 438 | 5905 | 186 | 10.0 | 20.6 | 3.27 | -1.8 |
| | | Height | | 141.7 | 365.4 | 1591.6 | 4153.7 | - | 164 | 9918 | 17 | | | | |
| Example 2 | Stretchable anisotropic cloth B | Width | 600 | 43.9 | 54.3 | 331.8 | - | - | 88 | 3726 | 57 | 159.1 | - | 4.36 | -1.4 |
| | | Height | | 6980.4 | - | - | - | - | 6 | 10718 | 2 | | | | |
| Comparative Example 1 | Stretchable isotropic cloth A | Width | 700 | 18.1 | 24.2 | 83.6 | 513.6 | 4128.0 | 375 | 14560 | 105 | 1.1 | 1.0 | 2.25 | -4.8 |
| | | Height | | 19.7 | 27.6 | 87.6 | 178.1 | 381.8 | 530 | 5905 | 249 | | | | |
| Comparative Example 2 | Stretchable isotropic cloth B | Width | 600 | 28.6 | 33.7 | 96.1 | 323.2 | 2505.6 | 430 | 14044 | 194 | | 1.4 | 2.82 | -3.3 |
| | | Height | | 35.9 | 43.9 | 133.8 | 269.0 | 651.1 | 472 | 5905 | 130 | | | | |
| Comparative Example 3 | Stretchable isotropic cloth C | Width | 350 | 28.6 | 35.5 | 143.9 | 561.4 | 3207.8 | 333 | 6839 | 102 | 1.1 | 1.0 | 2.99 | -5.8 |
| | | Height | | 30.4 | 37.0 | 151.0 | 501.2 | 2005.1 | 348 | 5117 | 110 | | | | |
| Comparative Example 4 | No cloth | | - | 8.5 | 15.5 | 49.2 | 76.5 | 120.5 | 360 | 216 | - | 1.0 | 1.0 | 2.54 | -1.5 |

From the results shown in Table 1, it became clear that sensitivity is improved by integrally forming the stretchable anisotropic cloth with the sensor body.

In addition, it became clear that if the cloth is a stretchable anisotropic cloth, stretchability of the sensor body is not inhibited and in the sensor sheet in which the stretchable anisotropic cloth is integrally formed with the sensor body, the same degree of hysteresis as that of the sensor body is maintained.

LIST OF REFERENCE SIGNS

| | |
|---|---|
| 1 | Sensor device |
| 2 | Sensor sheet |
| 3 | Measuring instrument |
| 3a | Schmitt trigger oscillator circuit |
| 3b | F/V conversion circuit |
| 4 | Display device |
| 4a | Monitor |
| 4b | Arithmetic circuit |
| 4c | Memory unit |
| 10, 40 | Sensor body |
| 11 | Dielectric layer (first dielectric layer) |
| 12A | Top electrode layer (first electrode layer) |
| 12B | Bottom electrode layer (second electrode layer) |
| 13A | Top conducting wire |
| 13B | Bottom conducting wire |
| 14A | Top connecting portion |
| 14B | Bottom connecting portion |
| 15A, 45A | Top protective layer |
| 15B, 45B | Bottom protective layer |
| 20A, 20B | Cloth |
| 21 | Non-stretchable member |
| 22A, 22B | Handle |
| 41A | First dielectric layer |
| 41B | Second dielectric layer |
| 42A | First electrode layer |
| 42B | Second electrode layer |
| 42C | Third electrode layer |
| 43A | First conducting wire |
| 43B | Second conducting wire |
| 43C | Third conducting wire |
| 44A | First connecting portion |
| 44B | Second connecting portion |
| 44C | Third connecting portion |

**Claims**

1.  A capacitive sensor sheet comprising:

    - a sensor body; and
    - a stretchable cloth integrally formed with the sensor body and having anisotropy in terms of stretchability,

    the sensor body comprising:

    - a sheet-like dielectric layer comprising an elastomer composition; and
    - a first electrode layer and a second electrode layer each comprising an electroconductive composition containing an electroconductive material,
    - wherein the first and the second electrode layers are formed on a top surface and a bottom surface of the dielectric layer respectively, and are at least partially opposed to each other across the dielectric layer,

wherein the at least partially opposed portions of the first and the second electrode layers constitute a detection portion, and

wherein the sensor body is reversibly deformable to change areas of the top and bottom surfaces of the dielectric layer.

2. The capacitive sensor sheet according to claim 1,
   wherein the cloth has a ratio of modulus at 5 % in a poorly-stretchable direction to modulus at 5 % in an easily-stretchable direction of 10 or more.

3. The capacitive sensor sheet according to claim 1 or 2,
   wherein the electroconductive material is at least carbon nanotubes.

4. The capacitive sensor sheet according to claim 1 or 2,
   wherein the elastomer composition contains urethane elastomer.

5. A sensor device comprising:

   - a capacitive sensor sheet according to any one of claims 1 to 4; and
   - a measuring instrument measuring a change in capacitance of the detection portion.

FIG. 1

FIG. 2A

FIG. 2B

FIG. 3A

FIG. 3B

27

FIG. 4A

FIG. 4B

FIG. 5

FIG. 6A

FIG. 6B

FIG. 6C

30

FIG. 7A

FIG. 7B

FIG. 7C

FIG. 7D

FIG. 8

EP 3 282 218 A1

FIG. 9

EP 3 282 218 A1

EP 3 282 218 A1

FIG. 10

FIG. 11

EP 3 282 218 A1

FIG. 12

FIG. 13

## INTERNATIONAL SEARCH REPORT

| International application No. |
| --- |
| PCT/JP2016/059954 |

| A. CLASSIFICATION OF SUBJECT MATTER |
| --- |
| *G01B7/16*(2006.01)i, *A61B5/11*(2006.01)i |
| According to International Patent Classification (IPC) or to both national classification and IPC |

| B. FIELDS SEARCHED |
| --- |
| Minimum documentation searched (classification system followed by classification symbols)<br>G01B7/16, A61B5/11 |

| Documentation searched other than minimum documentation to the extent that such documents are included in the fields searched |
| --- |
| Jitsuyo Shinan Koho  1922–1996 Jitsuyo Shinan Toroku Koho 1996–2016<br>Kokai Jitsuyo Shinan Koho 1971–2016 Toroku Jitsuyo Shinan Koho 1994–2016 |

| Electronic data base consulted during the international search (name of data base and, where practicable, search terms used) |
| --- |

### C. DOCUMENTS CONSIDERED TO BE RELEVANT

| Category* | Citation of document, with indication, where appropriate, of the relevant passages | Relevant to claim No. |
| --- | --- | --- |
| P,X | JP 2015-217127 A  (Sumitomo Riko Co., Ltd.),<br>07 December 2015 (07.12.2015),<br>paragraphs [0025] to [0075]; fig. 1 to 6<br>(Family: none) | 1-5 |
| A | JP 2014-113169 A  (Terumo Corp.),<br>26 June 2014 (26.06.2014),<br>paragraph [0025]; fig. 3<br>(Family: none) | 1-5 |
| A | JP 2009-172352 A  (Toyobo Co., Ltd.),<br>06 August 2009 (06.08.2009),<br>paragraphs [0029] to [0037]; fig. 1<br>(Family: none) | 1-5 |

☐ Further documents are listed in the continuation of Box C.    ☐ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 08 June 2016 (08.06.16) | 21 June 2016 (21.06.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
| --- | --- |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005096939 A

- JP 2005315831 A